# EUROPEAN PATENT APPLICATION

(11) **EP 4 589 019 A1**
(43) Date of publication of application: **23.07.2025**
(21) Application number: 23864481.9
(22) Date of filing: 19.07.2023
(51) Int. Cl.: C12Q 1/686, C12Q 1/6876

(54) **MULTIPLE-PCR REACTION SYSTEM**

(30) Priority: 14.09.2022 CN 202211120086
(71) Applicant: Maccura Biotechnology Co., Ltd., Chengdu Sichuan 611731 (CN)
(72) Inventor: GE, Zhiqi, Chengdu, Sichuan 611731 (CN); HU, Waner, Chengdu, Sichuan 611731 (CN); ZHAO, Yuhang, Chengdu, Sichuan 611731 (CN)
(74) Representative: Elzaburu S.L.P.
(86) International application number: PCT/CN2023/108202
(87) International publication number: WO 2024/055746

(57) **Abstract**

The present invention provides a design method of a single-tube ultra-multiplex PCR detection primer probe, a reaction system, and a multi-target detection kit based on the method and the reaction system.

## Description

### Field of the Invention

The present invention belongs to the field of molecular biology, in particular to the field of nucleic acid amplification and detection.

### Background of the Invention

Polymerase chain reaction (PCR) is a molecular biological technology that performs DNA replication via enzyme without using living organisms. The PCR is usually used in medical and biological research laboratories to undertake a variety of tasks, such as diagnosis of infectious diseases, gene cloning, phenotypic identification of laboratory animals, research on transcriptomes, detection of genetic diseases, identification of gene fingerprints, paternity test, etc. Since the PCR has an incomparable replication ability and accuracy, it is considered by the molecular biologists as a preferred method for nucleic acid detection. In the late 1990s, a real-time fluorescence quantitative PCR (qPCR) technology and related products launched by ABI in the USA further developed PCR to be a highly sensitive, highly specific and accurately quantitative nucleic acid sequence analysis technology.

At present, a primer probe design method that is widely used in qPCR platforms is mainly a TaqMan hydrolysis probe method, a working principle of which is mainly characterized by using an oligonucleotide probe that can specifically bind with a template and is labeled with a fluorescence group (donor) and a quencher group (receptor) at two ends thereof, and by designing a specific PCR primer upstream and downstream of the probe respectively. Before the start of PCR reaction, due to a principle of fluorescence resonance energy transfer (FRET), a fluorescence signal emitted by the fluorescence group at one end of the TaqMan probe is absorbed by the quencher group at the other end, such that the fluorescence signal cannot be detected by an instrument; but after the start of PCR amplification, the TaqMan probe specifically binds with the template, and when a DNA polymerase (taq polymerase) extends to a site at which the probe binds with the template, the TaqMan probe will be cleaved due to 5'-3' exonuclease activity of the DNA polymerase (taq polymerase), such that the fluorescence group labeled on the probe will be far away from the quencher group and a FRET structure cannot be formed any more, and thus a signal emitted by the fluorescence group can be detected by the instrument. However, if a PCR detection for multiplex targets is intended to be achieved in a same reaction system, it is required to arrange multiple TaqMan hydrolysis probes labeled with fluorescence groups of different wavelengths, and at present, only a detection of 4-6-plex different targets can be achieved at most due to limitation of a number of fluorescent channels in a detection instrument.

In order to achieve an ultra-multiplex PCR detection, in the prior art, the PCR detection technology is usually combined with other methods, for example, the PCR is combined with a hybrid chip to perform detection of multiplex targets with different amplification product lengths. For example, a patent document CN107090519A discloses a detection kit for common respiratory pathogens combining multiplex RT-PCR with a gene chip, in which, a principle of nucleic acid molecule hybridization is utilized, single-stranded probes targeting individual targets are arranged and fixed on the gene chip in a specific order to form a probe array, and then multiplex PCR products to be detected are allowed to be hybridized with it, such that target amplification products are hybridized with the probes on the chip so as to emit fluorescence signals. Although 20 types of respiratory pathogens can be simultaneously detected by this method, the gene chip is complex in preparation process, and is expensive, thereby being not conducive to clinical promotion. Moreover, the detection is cumbersome in procedures, requires multiple steps of uncovering and cleaning, and may easily cause contamination and result in a false positive result.

For another example, a patent document CN103074450B discloses a kit for simultaneous detection of thirty types of pathogenic bacteria causing diarrhea and a detection method thereof. The method combines the PCR amplification and capillary electrophoresis. In the method, PCR amplification products are taken out and subjected to capillary electrophoresis analysis; and the obtained spectrum is compared with a standard spectrum to determine the types of pathogenic bacteria causing diarrhea. In addition to the use of a PCR amplification detection equipment, this method further requires the use of a capillary electrophoresis equipment for product analysis, such that a detection cost is greatly increased, thereby being not conducive to clinical promotion and application either.

Therefore, there has been an urgent clinical need for a rapid, simple, and cost-effective multiplex nucleic acid detection method, applicable to diverse applications including but not limited to: detection of clinically common pathogenic microorganism, non-invasive screening for birth defect, single nucleotide polymorphism analysis, methylation analysis, genetic mutation analysis, and genotyping.

### Summary of the Invention

The problem to be solved by the present invention is to achieve detection and differentiation of multiple different targets in a single-tube reaction, and to avoid false positive signals generated by a primer dimer when multiple specific primers exist. The present invention provides a design method of a single-tube ultra-multiplex PCR detection primer probe, a reaction system, and a multi-target detection kit based on the method and the reaction system.

In a first aspect, the present invention provides a composition for PCR detection of a target sequence, comprising a detection probe; the detection probe comprising a detection sequence binding region, a detection group, and a blocking region; wherein
the detection sequence binding region is complementary to a detection sequence or a portion thereof,
the detection sequence binding region of the detection probe specifically binds to the detection sequence, followed by extending the detection sequence 0 or more nucleotides, to obtain an amplification product of the detection sequence,
the detection sequence binding region does not contain a sequence complementary or identical to the target sequence,
when the binding site of the detection probe to the detection sequence is located downstream of the blocking region, the blocking region blocks further extension of the detection sequence, and
a detection group on the detection probe emits different signals under the condition of hybridization between the detection probe with the detection sequence or the amplification product thereof compared to the condition of non-hybridization between the detection probe with the detection sequence or the amplification product thereof.

In some embodiments, the detection sequence does not contain the target sequence or a portion thereof, or a sequence complementary to the target sequence or a portion thereof.

The composition further comprises an auxiliary probe, the auxiliary probe comprising the detection sequence and a target specific sequence, wherein the target specific sequence comprises a sequence complementary to the target sequence or a portion thereof.

In some embodiments, the composition comprises n auxiliary probes and m detection probes for n target sequences, wherein 1 ≤ n ≤ 20, 1 ≤ m ≤ n.

Preferably, the n auxiliary probes contain detection sequences that are different from each other.

Preferably, each detection probe comprises a detection sequence binding region complementary to at least two types of detection sequences or a portion thereof and an extension sequence, and the m detection probes at least comprise detection sequence binding domains complementary to n detection sequences or a portion thereof. Under different detection probes, annealing temperatures of the hybrids formed by the amplification product and the detection probe may be identical. In some embodiments, the hybrid may be a duplex.

In some embodiments, the composition comprises n auxiliary probes and 1 detection probe for n target sequences, and the detection sequence binding region comprises ≥ n detection sequence binding domains for n detection sequences. In the case of one detection probe, annealing temperatures of the hybrids formed by different amplification products and the detection probe may be different. In some embodiments, the hybrid may be a duplex.

The individual detection sequence binding domains on the detection probe may be arranged in any order (for example, a first detection sequence binding domain may be located upstream (5' end) or downstream (3' end) of a second detection sequence binding domain). Similarly, other detection sequence binding domains may be arranged in any order (for example, as needed, a third detection sequence binding domain, a fourth detection sequence binding domain, a fifth detection sequence binding domain..., and a 20^{th} detection sequence binding domain).

The individual detection sequence binding domains on the detection probe may be arranged in any form, for example, the individual detection sequence binding domains on the detection probe may be arranged in a directly connected form, in a form connected via a connection sequence, or in a form with overlapping between the individual detection sequence binding domains. In some embodiments, the detection sequence binding domains are arranged in a form with partial or complete overlapping, wherein, when the detection sequence binding domains are arranged in a form with complete overlapping from each other, it is sufficient as long as there are several base mismatches between the corresponding detection sequence and the detection sequence binding domains.

The blocking region is a portion that is configured to prevent extension of the nucleic acid strand through DNA polymerase, thereby blocking strand extension during, for example, a PCR process. The blocking region may be Labelled with Spacer C3, Spacer C8 (Octanediol), Spacer 9/TEG, Spacer C12, Spacer 18/HEG, THF (tetrahydrofuran)/dSpacer (Abasic site), such that a corresponding site is blocked, so as to prevent extension reaction. The blocking region may also be a polymerase enzyme blocking group, which is a group that has a functional property of blocking further extension of a polymer. The blocking group may be any chemical group that can connect to a nucleotide, which allows a 5' end of a Labelled nucleotide to connect to a 3' end of another nucleotide in a DNA strand but does not allow a nucleotide to connect to a 3'hydroxyl group of the Labelled nucleotide. Properly, the absence of OH group at 3' position will prevent further extension by means of polymerase activity. In some embodiments, the blocking group is selected from acetyl, CH3, glycyl, leucyl, and alanyl groups. In some other embodiments, the blocking group may be in a form of a dipeptide or a tripeptide.

When the blocking region is located downstream of a binding site of the detection probe a to the detection sequence, after the detection probe binds with the detection sequence, the detection sequence continues to extend until it reaches a 5' end of the detection sequence.

In some embodiments, a part of detection sequence binding domains in the detection sequence binding region of the detection probe is located upstream of or across the blocking region, while another part of the detection sequence binding domains is located downstream of the blocking region, and after the detection sequence binds with the corresponding detection sequence binding domains of the detection probe, a portion of the detection sequence extends to a 5' end of the detection probe, and another portion thereof extends to the blocking region.

In some embodiments, all the detection sequence binding domains in the detection sequence binding region of the detection probe are located upstream of or across the blocking region, and after the detection sequence binds with the corresponding detection sequence binding domains of the detection probe, the detection sequence extends to the 5' end of the detection probe.

In some embodiments, all detection sequence binding domains in the detection sequence binding region of the detection probe are located downstream of the blocking region, and after the detection sequence binds with the corresponding detection sequence binding domains of the detection probe, the detection sequence extends to the blocking region of the detection probe.

In some embodiments, the detection probe is a linear detection probe or a detection probe with a hairpin structure.

In some embodiments, the composition further comprises n upstream oligonucleotide sequences for n target sequences respectively, wherein, the upstream oligonucleotide sequences each comprises a sequence complementary to a corresponding target sequence; and the upstream oligonucleotide sequences, after being hybridized with the target sequences, each is located at an upstream distal end of the auxiliary probe, or is located adjacent to an upstream of the auxiliary probe, or has an sequence partially overlapped with the target specific sequence of the auxiliary probe.

Preferably, the upstream oligonucleotide sequences are upstream primers specific to the target sequences or upstream probes specific to the target sequences.

The composition further comprises n downstream oligonucleotide sequences for n target sequences respectively, wherein the downstream oligonucleotide sequences each comprises a sequence complementary to a corresponding target sequence; and, when being hybridized with the target sequences, the downstream oligonucleotide sequences each is located downstream of the target specific sequence.

In some embodiments, all the upstream oligonucleotide sequences and all the downstream oligonucleotide sequences have an identical oligonucleotide sequence at 5' ends thereof, and the composition further comprises a universal primer having a sequence complementary to the identical oligonucleotide sequence.

In some embodiments, the detection probe has a length of 15-800 bases, 15-600 bases, 15-400 bases, or 15-160 bases.

In some embodiments, the detection sequence binding region of the detection probe has a length of 10-500 bases, 10-400 bases, or 10-100 bases.

In some embodiments, the detection probe is a self-quenching probe, and the detection group comprises a reporter group and a quencher group.

In some embodiments, the reporter group is a fluorescent group, and the quencher group is a molecule or group capable of absorbing/quenching the fluorescence.

In some embodiments, the detection sequence comprises the detection sequence comprises a target sequence or a portion thereof, or a sequence complementary to the target sequence or a portion thereof.

In some embodiments, the composition comprises a detection probe and a first primer set, wherein the first primer set comprises a first primer and a second primer that are each other's forward primer and reverse primer,
wherein, the detection probe comprises a first binding region A', a detection sequence binding region H, and a first blocking region; when the binding site of the detection probe to the detection sequence is located downstream of the first blocking region, the first blocking region blocks the extension of the detection sequence along the detection probe,
preferably, the first primer comprises a sequence A that is partially or completely complementary to the first binding region A' of the detection probe, and the second primer comprises a sequence h that is partially or completely identical to the detection sequence binding region H of the detection probe,
the first binding region A', the detection sequence binding region H, the sequence A and the sequence h each is not complementary or identical to the target sequence or any fragment thereof, and
preferably, a detection group on the detection probe is located downstream of the first blocking region. The detection group causes a signal change before and after hybridization between the detection probe and the detection sequence or between the detection probe and the amplification product of the detection sequence.

In some embodiments, when the blocking region is located downstream of the binding site of the detection probe to the detection sequence, after the detection probe binds with the detection sequence, the detection sequence continues to extend until it reaches the 5' end of the detection sequence.

In some embodiments, the detection probe comprises:
one detection sequence binding region H and one first binding region A' (A'-H; HA'); or
two detection sequence binding regions H and one first binding region A', the two detection sequence binding regions H being located on two sides of the first binding region A' respectively (H-A'-H); or
one detection sequence binding region H and two first binding regions A', the two first binding regions A' being located on two sides of the detection sequence binding region H respectively (A'-H-A'); or
two detection sequence binding regions H and two first binding regions A' that are arranged at intervals (H-A'-H-A' or A'-H-A'-H).

In some embodiments, the composition comprises m detection probes for n target sequences, and n first primer sets, wherein 1 ≤ n ≤ 20, and 1 ≤ m ≤ n.

In some embodiments, the m detection probes at least comprise detection sequence binding domains complementary to n detection sequences or a portion thereof. Under different detection probes, annealing temperatures of the hybrids formed by the amplification products and the detection probes may be the identical.

In some embodiments, the composition comprises one detection probe, which at least comprises detection sequence binding domains complementary to n detection sequences or a portion thereof. In the case of one detection probe, annealing temperatures of the hybrids formed by different amplification products and the detection probe are different. In some embodiments, the hybrid is a duplex.

The individual detection sequence binding domains may be arranged in a directly connected form, in a form connected via a connection sequence, or in a form with overlapping from each other. wherein, when the detection sequence binding domains are arranged in a form with complete overlapping from each other, it is sufficient as long as there are several base mismatches between the corresponding detection sequence and the detection sequence binding domains.

In some embodiments, the individual detection sequence binding domains on the detection probe may be arranged in any order (for example, a first detection sequence binding domain may be located upstream (5' end) or downstream (3' end) of a second detection sequence binding domain). Similarly, other detection sequence binding domains may be arranged in any order (for example, as needed, a third detection sequence binding domain, a fourth detection sequence binding domain, a fifth detection sequence binding domain..., and a 20^{th} detection sequence binding domain).

In some embodiments, the individual detection sequence binding domains on the detection probe may be arranged in any form, for example, the individual detection sequence binding domains on the detection probe may be arranged in a directly connected form, in a form connected via a connection sequence, or in a form with overlapping between the individual detection sequence binding domains. Preferably, the detection sequence binding domains are arranged in a form with overlapping. Wherein, when the detection sequence binding domains are arranged in a form with complete overlapping from each other, it is sufficient as long as there are several base mismatches between the corresponding detection sequence and the detection sequence binding domains.

In some embodiments, the detection probe is provided with a second blocking region at a 3' end thereof, wherein the second blocking region may prevent extension of the 3' end of the detection probe.

The blocking region is a portion that is configured to prevent extension of the nucleic acid strand through DNA polymerase, thereby blocking strand extension during, for example, a PCR process. The blocking region may be Labelled with Spacer C3, Spacer C8 (Octanediol), Spacer 9/TEG, Spacer C12, Spacer 18/HEG, THF (tetrahydrofuran)/dSpacer (Abasic site), such that a corresponding site is blocked, so as to prevent extension reaction. The blocking region may also be a polymerase enzyme blocking group, which is a group that has a functional property of blocking further extension of a polymer. The blocking group may be any chemical group that can connect to a nucleotide, which allows a 5' end of a Labelled nucleotide to connect to a 3' end of another nucleotide in a DNA strand but does not allow a nucleotide to connect to a 3'hydroxyl group of the Labelled nucleotide. Properly, the absence of OH group at 3' position will prevent further extension by means of polymerase activity. In some embodiments, the blocking group is selected from acetyl, CH3, glycyl, leucyl, and alanyl groups. In some other embodiments, the blocking group may be in a form of a dipeptide or a tripeptide.

In some embodiments, one single-stranded amplification product of the double-stranded amplification product of the first primer and the second primer is used as the detection sequence amplification product, which comprises the sequence A and a sequence h' that is partially or completely complementary to someone detection sequence binding domain of the detection sequence binding region H of the detection probe.

In some embodiments, the detection probe has a length of 15-800 bases, preferably, the detection probe has a length of 20-100 bases.

In some embodiments, the first binding region A' of the detection probe has a length of 5-65 bases, or 5-35 bases.

Preferably, the first binding region A' has a Tm value of 30°C-80°C, preferably 40°C-80°C.

Preferably, the first binding zone A' has a GC content of 30% -80%, preferably 40% -80%.

Preferably, the first binding region A' is designed at the 5' or 3' end of the detection probe, and is completely or partially complementary to the sequence A of the first primer.

Preferably, the detection probe is provided with the second blocking region at the 3' end thereof, and
the second blocking zone may prevent extension of the 3' end of the detection probe.

In some embodiments, the detection sequence binding region H has a length of 10-500 bases, 10-400 bases, or 10-100 bases.

In some embodiments, the detection sequence binding domain has a length of 5-65 bases.

Preferably, the detection sequence binding domain has a Tm value of 30°C-80°C, preferably 40°C-80°C.

Preferably, the detection sequence binding domain has a GC content of 20% -80%, preferably 40% -80%.

In some embodiments, the detection group comprises a first detection group and a second detection group, wherein the first detection group is a fluorescence reporter group, and the second detection group is a quencher group or other labelling groups that can produce a signal change with the first detection group through fluorescence resonance energy transfer.

Preferably, the second detection group is spaced apart from the first detection group by 3-140 angstroms.

Preferably, the first detection group and the second detection group are both located on the first binding region A', or are both located on the detection sequence binding region H, or are located on the first binding region A' and the detection sequence binding region H, respectively.

In some embodiments, the first primer further comprises a target sequence binding region, and the second primer further comprises a target sequence binding region.

Preferably, the first primer sequentially comprises the sequence A and the target sequence binding region from 5' end to 3' end.

Preferably, the target sequence binding region is a target specific sequence that specifically binds with a target sequence to be amplified.

In some embodiments, an annealing temperature between the detection probe and the first primer is TmA, an annealing temperature between the detection probe and a complementary sequence of the second primer is TmH, an annealing temperature between the first primer and the target sequence is Tmb1, and an annealing temperature between the second primer and the target sequence is Tmb2. In some specific embodiments, TmA<Tmb1 and TmA<Tmb2, and/or TmH<Tmb1 and TmH<Tmb2.

In some embodiments, the first binding region A' is complementary to the sequence A of the first primer.

Preferably, the sequence A of the first primer is a freely designed sequence that does not bind with the target sequence or any fragment thereof in a paired manner, and is partially or completely complementary to the first binding region A' of the detection probe.

Preferably, there are 0-20 bases spaced between the sequence A and the target sequence binding region of the first primer.

Preferably, the first primer has a length of 20-80 bases.

Preferably, the target sequence binding region of the first primer has a Tm value of 40°C-80°C.

Preferably, target sequence binding region of the first primer has a GC content of 20%-80%.

Preferably, the sequence A of the first primer has a length of 5-65 bases, or 5-35 bases.

Preferably, the sequence A of the first primer has a Tm value of 40°C-80°C.

Preferably, the sequence A of the first primer has a GC content of 30%-80%.

In some embodiments, someone detection sequence binding domain of the detection sequence binding region H and the sequence h of the second primer have a partially or completely identical sequence.

In some embodiments, the second primer sequentially comprises the sequence h and the target sequence binding region from 5' end to 3' end.

Preferably, the target sequence binding region is a target specific sequence, and specifically binds with a target sequence to be amplified. Preferably, the sequence h of the second primer has a GC content of 20%-80%.

In some embodiments, when there is no target to be amplified, the first binding region A' of the detection probe completely or partially binds with the sequence A of the first primer in a complementary manner, and the other part of the detection probe is in a single-stranded state. The single-stranded flexible detection probe is formed as a curly shape due to molecular flexibility or is formed as a folded shape due to a secondary structure, at this time, a distance between the first detection group and the second detection group is relatively close, and the efficiency of fluorescence resonance energy transfer is relatively high.

In some embodiments, during PCR amplification, the first primer and the second primer specifically bind with the target sequence respectively and extend to produce a double-stranded pre-amplification product. A first single-stranded pre-amplification product (S1) of the double-stranded pre-amplification product is used as the detection sequence which comprises the sequence A, the target sequence, and a complementary sequence h' of the sequence h. The detection sequence binding region H of the detection probe is complementarily paired with the complementary sequence h' of the sequence h of the first single-stranded pre-amplification product, the first binding region A' of the detection probe is complementarily paired with the sequence A of the first single-stranded pre-amplification product, and the 3' end of the first single-stranded pre-amplification product as the detection sequence may continue to extend by 0 or more bases to the 5' end or the first blocking region of the detection probe (P), to obtain an amplification product of the detection probe (P) which is a partially or completely reverse complementary sequence of the detection probe (P) and the detection sequence, that is, a secondary amplification in which the single-stranded pre-amplification product (S1) is used as a primer and the detection probe (P) is used as a template is completed, so as to obtain a double-stranded secondary amplification product.

The detection probe is changed from a single-stranded state to a double-stranded state, and a distance between the first detection group and the second detection group is increased, thereby generating a signal that may be detected by an instrument.

In some embodiments, a target sequence amplification is identified by means of a fluorescence channel, or a target sequence amplification is identified by means of a melting temperature curve, or a target sequence amplification is identified by means of a fluorescence channel + a melting temperature curve.

Preferably, a target sequence amplification is identified by means of the melting temperature curve. Specifically, after PCR amplification, a melting curve analysis is performed, during a process of temperature change, the double-stranded product formed by the detection probe and the amplification product of the detection sequence will melt at a certain temperature, at this time, the detection probe with the first detection group and the second detection group will be changed from a double strand to a single strand, such that the distance between the detection groups is changed, thereby generating a signal that may be detected by the instrument.

Due to the fact that a melting temperature of the double-stranded amplification product formed by the detection probe (P) and the detection sequence is detected during melting curve analysis, it is possible to adjust the melting temperature of the double-stranded amplification product formed by the detection probe (P) and the detection sequence by adjusting a sequence and length of the sequence h of the reverse primer (R) to obtain the single-stranded pre-amplification product (S1) that is partially or completely reverse complementary to the detection sequence binding domain (H) of the detection probe (P). For example, it is possible to increase or decrease the melting temperature of the double-stranded amplification product formed by the detection probe (P) and the detection sequence by adjusting a length or sequence of the sequence h, or adjusting a position at which the sequence h is reversely complementary to the detection sequence binding domain of the detection sequence binding region (H) of the detection probe (P).

In some embodiments, the detection probe-primer set comprises n first primer sets and m detection probes for n target sequences, wherein 1 ≤ n ≤ 20, and 1 ≤ m ≤ n.

The base sequences of different detection probes are different, and the detection groups on the detection probes are different,

The m detection probes at least comprise detection sequence binding domains complementary to n detection sequences or a portion thereof.

The annealing temperatures of the hybrids formed by detection sequence amplification products (obtained by amplifying different target sequences with all the first primer sets for the same detection probe) and the detection probe are different from each other. In some embodiments, the hybrid is a duplex.

Preferably, the annealing temperatures of the hybrids formed by detection sequence amplification products (obtained by amplifying different target sequences with all the first primer sets for the same detection probe) and the detection probe are different from each other by a △Tm of 4-12°C. In some embodiments, the hybrid is a duplex.

Preferably, target sequence binding regions included in all the first primers are different from each other, and/or target sequence binding regions included in all the second primers are different from each other.

In some embodiments, the detection probe-primer set comprises n first primer sets for and m detection probes n target sequences, wherein 1 ≤ n ≤ 20, and 1 ≤ m ≤ n,

The m detection probes comprise detection sequence binding domains complementary to n detection sequences or a portion thereof.

In some embodiments, the detection probe-primer set comprises n first primer sets and one detection probe for n target sequences, wherein 1 ≤ n ≤ 20.

The detection probe comprises detection sequence binding domains complementary to n detection sequences or a portion thereof.

The individual detection sequence binding domains may be arranged in a directly connected form, in a form connected via a connection sequence, or in a form with partial or complete overlapping. wherein, when the detection sequence binding domains are arranged in a form with complete overlapping from each other, it is sufficient as long as there are several base mismatches between the corresponding detection sequence and the detection sequence binding domains.

In some embodiments, n detection probes are designed for n target sequences, and then an identification of amplifications of n target sequences is carried out by using a fluorescence channel. In such embodiments, the n detection probes are flexible detection probes.

The flexible detection probe forms a curly shape due to molecular flexibility or forms a folded shape due to a secondary structure in single-stranded state, but after it is extended to be double-stranded state by hybridization or annealing, it is fixed to form a relatively rigid double-helix structure due to hydrogen-bond interaction. The flexible detection probe may be selected from a group consisting of: an oligonucleotide that forms a curly shape due to molecular flexibility in single-stranded state, an oligonucleotide that can form a hairpin structure in single-stranded state, an oligonucleotide that can form a stem-loop structure in single-stranded state, an oligonucleotide that can form a pseudoknot structure in single-stranded state, and an oligonucleotide that can form a triplex structure in single-stranded state.

In some embodiments, only one universal detection probe or several universal detection probes are designed for n target sequences, and then amplifications of n target sequences are identified by using melting curves. In such embodiments, a sequence and/or length of the sequence h of the second primer is adjusted to obtain the complementary sequence of the sequence h of the first single-stranded pre-amplification product that is partially or completely complementary to the corresponding detection sequence binding domains of the detection sequence binding region H of the detection probe, and/or a sequence and/or length of the sequence A of the first primer is adjusted to obtain the complementary sequence of the sequence A of the first single-stranded pre-amplification product that is partially or completely complementary to the first binding region A' of the detection probe, such that a melting temperature of the double-stranded product formed by the detection probe and the first single-stranded amplification product is adjusted.

When there is no target sequence to be detected, the first binding region (A') of the detection probe (P) is reversely complementary to the sequence A of the forward primer (F), and the other part of the detection probe (P) is in a single-stranded state. However, when a target sequence to be detected is present, the forward primer (F) and the reverse primer (R) specifically bind with the target sequence respectively and extend to produce a double-stranded pre-amplification product; the corresponding detection sequence binding domains of the detection sequence binding region (H) at the 3' end of the detection probe (P) are complementarily paired with the reverse complementary sequence (h') of the sequence h at the 3' end of the single-stranded pre-amplification product (S1); the first binding region (A') at the 5' end of the detection probe is complementarily paired with the sequence A of the single-stranded pre-amplification product (S1) (which is the sequence A at the 5' end of the detection sequence); and the 3' end of the single-stranded pre-amplification product (S1) (i.e. the detection sequence), may continue to extend by 0 or more bases to the 5' end or first blocking region of the detection probe (P), to obtain a partially or completely reverse complementary sequence of the detection probe (P); that is, a secondary amplification in which the sequence h' of the single-stranded pre-amplification product (S1) binding with the detection sequence binding region of the detection probe P is used as a primer and the detection probe (P) is used as a template is completed, so as to obtain a double-stranded secondary amplification product of the detection probe (P) and the detection sequence; at this time, the distance between the first detection group and the second detection group is increased due to a partial or complete change of the detection probe from the single strand to the double strand, and the efficiency of fluorescence resonance energy transfer is decreased, such that a fluorescence signal change is produced and may be detected by an instrument. After the completion of PCR, when the melting curve analysis is performed, the double-stranded secondary amplification product formed by the detection probe (P) and the detection sequence will melt at a certain temperature, such that the detection probe (P) with the first detection group and the second detection group becomes a single-stranded state, at this time, the distance between the first detection group and the second detection group is decreased, and the efficiency of fluorescence resonance energy transfer is increased, such that a fluorescence signal change is produced again, which may be used for melting curve analysis.

Preferably, it is possible to increase or decrease the melting temperature of the double-stranded product formed by the detection probe and the detection sequence by adjusting a length or sequence of the sequence A and/or the sequence h, or adjusting the complementary position of the complementary sequence of the sequence A and/or the sequence h with the first binding region A' and/or the detection sequence binding domains of the detection sequence binding region (H) of the detection probe (P).

Such embodiments can achieve an ultra-multiplex PCR or a digital PCR in a single tube, and can effectively avoid the formation of primer dimers.

In the second aspect, the present invention provides a reaction system for PCR detection, comprising the composition of the first aspect.

In a third aspect, the present invention provides a detection kit for PCR detection, comprising the composition of the first aspect or the reaction system of the second aspect.

In a fourth aspect, the present invention provides a method for PCR detection, comprising the step of performing PCR by using the composition of the first aspect, the reaction system of the second aspect, or the detection kit of the third aspect.

In some embodiments, the method comprises the following steps:
(1) reacting, under a condition that allows for hybridization, a detection sample with the upstream oligonucleotide sequence and the auxiliary probe;
(2) reacting, under a condition that allows for cleavage of the auxiliary probe, a product obtained in step (1) with an enzyme having 5' endonuclease activity and/or 5' exonuclease activity;
(3) reacting, under a condition that allows for hybridization, a product obtained in step (2) with the detection probe;
(4) reacting, under a condition that allows for extension reaction of a nucleic acid polymerase, a product obtained in step (3) with the nucleic acid polymerase;
(5) performing melting curve analysis to a product obtained in step (4); and determining the presence or absence of target sequence in the detection sample, based on a result of the melting curve analysis.

Preferably, under the condition that allows for nucleic acid hybridization, the detection sample is allowed to react with the upstream oligonucleotide sequence, the auxiliary probe, and the downstream oligonucleotide sequence; such that, the target sequence is used as a template, and the upstream oligonucleotide sequence and downstream oligonucleotide sequence are used as an upstream primer and a downstream primer respectively, for amplification, generating the target nucleic acid sequence, thereby improving the sensitivity of the method.

In some embodiments, during the detection process, n upstream primers, n downstream primers, and n auxiliary probes are hybridized (annealed) with corresponding n target sequences, respectively; subsequently, under action of the nucleic acid polymerase, all the upstream primers and downstream primers are extended respectively, and the extension of the upstream primers (forward primers) results in that corresponding auxiliary probe (auxiliary probe) are cleaved by the enzyme having 5' endonuclease activity and/or 5' exonuclease activity, so as to release auxiliary fragments; then, the auxiliary fragments are hybridized to different positions of the detection probe and are extended by the nucleic acid polymerase, so as to obtain n extension products; and the n extension products have different lengths and, , together with the detection probes, form n hybrids with different Tm values. Whereby, through melting curve analysis, the presence of hybrids with specific Tm values may be determined, and then the presence of target nucleic acid molecules corresponding to the hybrids may be further determined. In some embodiments, the hybrid is a duplex.

Therefore, in the method of the present invention, one detection probe and n auxiliary probes may be used to achieve the detection of n target nucleic acid molecules.

In some embodiments, a first primer set is utilized to specifically bind with the target sequence and extend to produce a double-stranded pre-amplification product, wherein one single-stranded amplification product of the double-stranded pre-amplification products comprises the sequence A, the target sequence, and a complementary sequence h' of the sequence h, and serves as the detection sequence; and
after the A and h' of the single-stranded amplification product bind specifically to the probe, the resultant is subjected to amplification, and a signal change that may detected by an instrument is produced. The presence of the target sequence is determined according to the signal change.

In some embodiments, during PCR amplification, the first primer and the second primer specifically bind with the target sequence respectively and extend to produce a double-stranded pre-amplification product. A first single-stranded pre-amplification product (S1) of the double-stranded pre-amplification product is used as the detection sequence which comprises the sequence A, the target sequence, and a complementary sequence h' of the sequence h; and the detection sequence binding region H of the detection probe is complementarily paired with the complementary sequence h' of the sequence h of the first single-stranded pre-amplification product, and the first binding region A' of the detection probe is complementarily paired with the sequence A of the first single-stranded pre-amplification product. The 3' end of the first single-stranded pre-amplification product as the detection sequence may continue to extend by 0 or more bases to the 5' end or the first blocking region of the detection probe (P), to obtain an amplification product of the detection probe (P) which is a partially or completely reverse complementary sequence of the detection probe (P) and the detection sequence, that is, a secondary amplification in which the sequence h' at which the single-stranded pre-amplification product (S1) binds with the detection sequence binding region of the detection probe is used as a primer and the detection probe (P) is used as a template is completed, so as to obtain a double-stranded secondary amplification product.

The detection probe is changed from a single-stranded state to a double-stranded state, and a distance between the first detection group and the second detection group is increased, thereby generating a signal that may be detected by an instrument.

When there is no target sequence to be detected, the first binding region (A') of the detection probe (P) is reversely complementary to the sequence A of the forward primer (F), and the other part of the detection probe (P) is in a single-stranded state. However, when a target sequence to be detected is present, the forward primer (F) and the reverse primer (R) specifically bind with the target sequence respectively and extend to produce a double-stranded pre-amplification product; the corresponding detection sequence binding domains of the detection sequence binding region (H) at the 3' end of the detection probe (P) are complementarily paired with the reverse complementary sequence (h') of the sequence h at the 3' end of the single-stranded pre-amplification product (S1); the first binding region (A') at the 5' end of the detection probe is complementarily paired with the sequence A of the single-stranded pre-amplification product (S1) (which is the sequence A at the 5' end of the detection sequence); and the 3' end of the single-stranded pre-amplification product (S1) may continue to extend by 0 or more bases to the 5' end or first blocking region of the detection probe (P), to obtain a partially or completely reverse complementary sequence of the detection probe (P); that is, a secondary amplification in which the sequence h' of the single-stranded pre-amplification product (S1) binding with the detection sequence binding region of the detection probe is used as a primer and the detection probe (P) is used as a template is completed, so as to obtain a double-stranded secondary amplification product of the detection probe (P) and the detection sequence; at this time, the distance between the first detection group and the second detection group is increased due to a partial or complete change of the detection probe from the single strand to the double strand, and the efficiency of fluorescence resonance energy transfer is decreased, such that a fluorescence signal change is produced and may be detected by an instrument. After the completion of PCR, when the melting curve analysis is performed, the double-stranded secondary amplification product formed by the detection probe (P) and the detection sequence will melt at a certain temperature, such that the detection probe (P) with the first detection group and the second detection group becomes a single-stranded state, at this time, the distance between the first detection group and the second detection group is decreased, and the efficiency of fluorescence resonance energy transfer is increased, such that a fluorescence signal change is produced again, which may be used for melting curve analysis.

In some embodiments, a target sequence amplification is identified by means of a fluorescence channel, or a target sequence amplification is identified by means of a melting temperature curve, or a target sequence amplification is identified by means of a fluorescence channel + a melting temperature curve.

In a fifth aspect, the present invention provides use of the composition of the first aspect, the reaction system of the second aspect, or the detection kit of the third aspect in PCR detection.

In particular, the present invention provides use of the composition of the first aspect, the reaction system of the second aspect, or the detection kit of the third aspect in single-tube ultra-multiplex PCR detection.

In a sixth aspect, the present invention provides use of the composition of the first aspect, the reaction system of the second aspect, the detection kit of the third aspect, or the method of the fourth aspect in detection of clinically common pathogenic microorganism, non-invasive screening for birth defect, single nucleotide polymorphism analysis, methylation analysis, genetic mutation analysis, or genotyping.

### Brief Description of the Drawings

Fig. 1 shows that there is no melting peak for a no-template control, while a melting peak with a Tm value of 59°C is formed in a ROX channel for a positive reaction well added with HPV type 45 plasmids.
Fig. 2 shows that there is no melting peak for a no-template control, while a melting peak with a Tm value of 80°C is formed in a FAM channel for a positive reaction well added with HPV type 16 plasmids.
Fig. 3 shows that there is no melting peak for a no-template control, while melting peaks with the Tm values of 80.2°C, 75°C, 69.4°C, and 59.4°C are formed respectively in a ROX channel for positive reaction wells added with HPV types 16, 31, 35, and 45 plasmids.
Fig. 4 shows that there is no melting peak for a no-template control, while a melting peak with a Tm value of 67.62°C is formed in a CY5 channel for a positive sample reaction well added with non-small cell lung cancer EGFR exon 20 H773_V774insH mutation (F6 and R7 are used as the first primer set).
Fig. 5 shows that there is no melting peak for a no-template control, while a melting peak with a Tm value of 74.17°C is formed in a CY5 channel for a positive sample reaction well added with non-small cell lung cancer EGFR exon 20 H773_V774insH mutation (F6 and R7 are used as the first primer set).
Fig. 6 is a schematic diagram of an embodiment.
Fig. 7 is a schematic diagram of another embodiment.
Fig. 8 shows a result of multiplex detection by utilizing a probe comprising a blocking region.
Fig. 9 shows a result of multiplex detection by utilizing a probe comprising no blocking region.

### Detailed Description of the Embodiments

Preferred embodiments of the present invention are described in the following, but the protection scope of the present invention is not limited to the following preferred embodiments. It should be noted that, several variations and improvements made by those skilled in the art based on the concept of the present invention are within the protection scope of the present invention.

Herein, the terms "target sequence", "target nucleic acid", "target nucleic acid sequence", or "target" may be used interchangeably and mean a portion of the nucleic acid sequence to be amplified, to be detected, or to be amplified and detected, which may be annealed with a primer under annealing or amplification conditions.

Herein, the term "upstream primer", also known as forward primer, is an oligonucleotide that extends continuously along a negative strand; and the term "downstream primer" used in the present invention, also known as reverse primer, is an oligonucleotide that is continuously extended along a positive strand, wherein, the positive strand, i.e. a sense strand, also known as a positive-sense strand or a coding strand, is generally located at an upper end of a double-stranded DNA, with a direction of 5'-3' from left to right, a base sequence thereof is substantially the same as a gene mRNA, and a primer binding with this strand is a reverse primer; and the negative strand, i.e. a nonsense strand, also known as a non-coding chain, is complementary to the positive strand, and a primer binding with this strand is a forward primer. It should be understood that, when the designations of the sense strand and the antisense strand are interchanged, the nominations of corresponding forward primer and reverse primer may also be interchanged accordingly.

Herein, the term "probe" refers to a labeled oligonucleotide for detecting whether a target is present.

Herein, the term "base-complementary pairing" refers to a phenomenon where A and T, A and U, and G and C, etc., in a corresponding relationship, are connected to each other via a hydrogen bond. Correspondingly, "base mismatching" refers to all other pairing situations except for those specified in "base-complementary pairing", such as a mismatching of A and C, A and G, T and G, or T and C.

The blocking region is namely a portion that is configured to prevent extension of the nucleic acid strand through DNA polymerase, thereby blocking strand extension during, for example, a PCR process. The blocking region may be Labelled with Spacer C3, Spacer C8 (Octanediol), Spacer 9/TEG, Spacer C12, Spacer 18/HEG, THF (tetrahydrofuran)/dSpacer (Abasic site), such that a corresponding site is blocked, so as to prevent extension reaction. The blocking region may also be a polymerase enzyme blocking group, which is a group that has a functional property of blocking further extension of a polymer. The blocking group may be any chemical group that can connect to a nucleotide, which allows a 5' end of a Labelled nucleotide to connect to a 3' end of another nucleotide in a DNA strand but does not allow a nucleotide to connect to a 3'hydroxyl group of the Labelled nucleotide. Properly, the absence of OH group at 3' position will prevent further extension by means of polymerase activity. In some embodiments, the blocking group is selected from quencher groups (such as BHQ, MGB, etc.), acetyl, CH3, glycyl, leucyl, and alanyl groups. In some other embodiments, the blocking group may be in a form of a dipeptide or a tripeptide.

### Embodiment 1:

In the method of the present invention, a detection probe and auxiliary probes are used to detect target nucleic acid molecules. In this embodiment, a self-quenching detection probe (with a fluorescent group and a quencher group) is provided, and one upstream primer, one downstream primer, and one auxiliary probe are designed and provided for each target nucleic acid molecule; wherein, the auxiliary probe comprises a unique detection sequence that can hybridize with the detection probe. The hybridization positions between different detection sequences and the detection probe are unique, but there may be an overlapping region therebetween. During the detection process, the upstream primers, the downstream primers and the auxiliary probes are hybridized (annealed) with corresponding target sequences, respectively; subsequently, under action of the nucleic acid polymerase, the upstream primers and downstream primers are extended respectively, and the extension of the upstream primers results in that corresponding auxiliary probes are cleaved by the enzyme having 5' endonuclease activity and/or 5' exonuclease activity, so as to release detection sequences; then, the detection sequences are hybridized onto the detection probe and are extended by the nucleic acid polymerase, so as to obtain extension products; and different extension products and the detection probe form hybrids with different Tm values. Whereby, through melting curve analysis, the presence of hybrids with specific Tm values may be determined, and then the presence of target nucleic acid molecules corresponding to the hybrids may be further determined. In some embodiments, the hybrid is a duplex.

The reaction system comprises the following steps:
(1) providing one detection probe, and providing one forward primer, one auxiliary probe, and one reverse primer for each target nucleic acid sequence to be detected;
(2) mixing a sample to be detected with the provided detection probe, upstream primer, auxiliary probe, downstream primer, and a DNA polymerase;
(3) incubating the product obtained in step (2) under a condition that allow nucleic acid denaturation;
(4) incubating the product obtained in step (3) under a condition that allow nucleic acid annealing or hybridization;
(5) incubating the product obtained in step (4) under a condition that allow nucleic acid extension; and
(6) optionally, repeating steps (3)-(5) for one or more times.
(7) performing melting curve analysis after the PCR amplification is completed, wherein, the double-stranded product formed by the detection probe and the amplification product of the detection sequence will melt at a certain temperature, at this time, the detection probe with the first detection group and the second detection group will be changed to a single strand, such that a fluorescence signal change is produced, which may be detected by an instrument.

Preferably, the reaction conditions described in the present invention are as follows: performing pre-denaturation at 90°C-96°C for 2-15 minutes; performing denaturation at 90°C-95°C for 10-60 seconds, and performing annealing and extension at 50°C-75°C for 30-90 seconds, for 35-50 cycles; and performing melting curve analysis at 35-95°C with a heating rate of 0.05°C/s, and collecting fluorescence signals.

More preferably, the reaction conditions described in the present invention are as follows: performing pre-denaturation at 95°C for 3 minutes; performing denaturation at 94°C for 10 seconds, and performing annealing and extension at 56°C for 30 seconds, for a total of 45 cycles; and performing melting curve analysis at 40-95°C with a heating rate of 0.05°C/S, and collecting fluorescence signals.

Preferably, concentrations of the primers and probe in a reaction system are as follows respectively: the forward primer has a concentration of 30 nM-1000 nM, the reverse primer has a concentration of 30 nM-500 nM, the auxiliary probe has a concentration of 150 nM-1200 nM, and the detection probe has a concentration of 150 nM-1200 nM.

### Embodiment 2:

During specific amplification of a pathogenic target, the forward primer (F2) and the reverse primer (R2) amplify a target nucleic acid to obtain a double-stranded pre-amplification product, wherein, one single-stranded pre-amplification product (S1) sequentially comprises, from 5' end to 3' end, the sequence A, the target sequence, and the reverse complementary sequence h' of the corresponding detection sequence binding region of the detection sequence binding region. The corresponding detection sequence binding domains of the detection sequence binding region (H) of the detection probe (P1) are complementarily paired with the reverse complementary sequence (h') of the sequence h of the one single-stranded pre-amplification product (S1); the first binding region (A') of the detection probe (P1) is complementarily paired with the sequence A at the 5' end of the single-stranded pre-amplification product (S1). The 3' end of the single-stranded pre-amplification product (S1) may continue to extend to the first blocking region of the detection probe P1, to obtain a partially or completely reverse complementary sequence of the detection probe P1, that is, a secondary amplification in which the single-stranded pre-amplification product (S1) is used as a primer and the detection probe P1 is used as a template is completed, so as to obtain a secondary amplification product of the detection probe P1 and the detection sequence. After the PCR amplification is completed, a melting curve analysis is performed, wherein, during a process of temperature change, the secondary amplification product of the detection probe P1 will melt at a certain temperature, at this time, the distance between the first detection group and the second detection group is changed, such that a signal change is produced, which may be detected by an instrument.

The reaction system involves the following steps:
(1) Firstly, in a cycle of PCR amplification reaction, the forward primer (F2) and the reverse primer (R2) may specifically amplify a target sequence. After amplification, a double-stranded pre-amplification product will be obtained, wherein, one single-stranded pre-amplification product (S1) sequentially comprises, from 5' end to 3' end, the sequence A, the target sequence, and the reverse complementary sequence h' of the corresponding detection sequence binding region of the detection sequence binding region. The corresponding detection sequence binding domains of the detection sequence binding region (H) of the detection probe (P1) are complementarily paired with the reverse complementary sequence (h') of the sequence h of the one single-stranded pre-amplification product (S1); and the first binding region A' of the detection probe (P1) is complementarily paired with the sequence A at the 5' end of the single-stranded pre-amplification product (S1). The 3' end of the single-stranded pre-amplification product (S1) may continue to extend to the first blocking region of the detection probe (P1), to obtain a partially or completely reverse complementary sequence of the detection probe (P1), that is, a secondary amplification in which the single-stranded pre-amplification product (S1) is used as a primer and the detection probe (P2) is used as a template is completed, so as to obtain a secondary amplification product of the detection probe (P1).
(2) After the PCR amplification is completed, a melting curve analysis is performed, wherein, during a process of temperature change, the secondary amplification product of the detection probe (P1) will melt at a certain temperature, at this time, the distance between the first detection group and the second detection group is changed, such that a signal change is produced, which may be detected by an instrument.

Preferably, the reaction conditions are as follows: performing pre-denaturation at 90°C-96°C for 2-15 minutes; performing denaturation at 90°C-95°C for 10-60 seconds, and performing annealing and extension at 50°C-75°C for 30-90 seconds, for 35-50 cycles; and performing melting curve analysis at 35-95°C with a heating rate of 0.05°C/s, and collecting fluorescence signals.

More preferably, the reaction conditions are as follows: performing pre-denaturation at 95°C for 3 minutes; performing denaturation at 94°C for 10 seconds, and performing annealing and extension at 56°C for 30 seconds, for a total of 45 cycles; and performing melting curve analysis at 40-95°C with a heating rate of 0.05°C/S, and collecting fluorescence signals.

Preferably, concentrations of the primers and probe in a reaction system are as follows respectively: the forward primer (F2) has a concentration of 30 nM-1000 nM, the reverse primer (R2) has a concentration of 30 nM-500 nM, and the detection probe (P1) has a concentration of 150 nM-1200 nM.

### Embodiment 3:

During specific amplification of a target sequence, the forward primer (F) and the reverse primer (R) amplify a target nucleic acid to obtain a double-stranded pre-amplification product, wherein, one single-stranded pre-amplification product (S1) sequentially comprises, from 5' end to 3' end, the sequence A, the target sequence, and the reverse complementary sequence h' of the corresponding detection sequence binding region of the detection sequence binding region. The corresponding detection sequence binding domains of the detection sequence binding region (H) of the detection probe (P1) are complementarily paired with the reverse complementary sequence (h') of the sequence h of the one single-stranded pre-amplification product (S1); the first binding region (A') of the detection prob is complementarily paired with the sequence A at the 5' end of the single-stranded pre-amplification product (S1). The 3' end of the single-stranded pre-amplification product (S1) may continue to extend or may be directly hybridized to the first blocking region of the detection probe (P1), to obtain a partially or completely reverse complementary sequence of the detection probe (P1), that is, a secondary amplification in which the single-stranded pre-amplification product (S1) is used as a primer and the probe (P1) is used as a template is completed, so as to obtain an amplification product of the detection sequence.

After the PCR amplification is completed, a melting curve analysis is performed, wherein, during a process of temperature change, the secondary amplification product of the detection probe (P1) will melt at a certain temperature, at this time, the distance between the first detection group and the second detection group is changed. Since the melting temperatures of the secondary amplification products having different lengths are different, different melting peaks may be formed.

The reaction system involves the following steps:
(1) Firstly, in a cycle of PCR amplification reaction, the forward primer (F) and the reverse primer (R) may specifically amplify a target nucleic acid sequence. After amplification, a double-stranded pre-amplification product will be obtained, wherein, one single-stranded pre-amplification product (S1) sequentially comprises, from 5' end to 3' end, the sequence A, the target sequence, and the reverse complementary sequence h' of the sequence h. The corresponding detection sequence binding domains of the detection sequence binding region (H) of the detection probe (P1) are complementarily paired with the reverse complementary sequence (h') of the sequence h of the one single-stranded pre-amplification product (S1); and the first binding region A' of the detection probe (P1) is complementarily paired with the sequence A at the 5' end of the single-stranded pre-amplification product (S1). The 3' end of the single-stranded pre-amplification product (S1) may continue to extend by o or more bases to the first blocking region or the 5' end of the detection probe (P1), to obtain a partially or completely reverse complementary sequence of the detection probe (P1), that is, a secondary amplification in which the single-stranded pre-amplification product (S1) is used as a primer and the detection probe (P1) is used as a template is completed, so as to obtain an amplification product of the detection sequence.
(2) After the PCR amplification is completed, a melting curve analysis is performed, wherein, during a process of temperature change, the secondary amplification product of the detection probe (P1) will melt at a certain temperature, at this time, the distance between the first detection group and the second detection group is changed. Since the melting temperatures of the secondary amplification products having different lengths are different, different melting peaks may be formed.

Preferably, the reaction conditions are as follows: performing pre-denaturation at 90°C-96°C for 2-15 minutes; performing denaturation at 90°C-95°C for 10-60 seconds, and performing annealing and extension at 50°C-75°C for 30-90 seconds, for 35-50 cycles; optionally, performing incubation at a constant temperature of 40°C for 15 seconds, for 5 cycles; and performing melting curve analysis at 35-95°C with a heating rate of 0.05°C/s, and collecting fluorescence signals.

More preferably, the reaction conditions are as follows: performing pre-denaturation at 95°C for 3 minutes; performing denaturation at 94°C for 10 seconds, and performing annealing and extension at 56°C for 30 seconds, for a total of 45 cycles; optionally, performing incubation at a constant temperature of 40°C for 15 seconds, for 5 cycles; and performing melting curve analysis at 40-95°C with a heating rate of 0.05°C/S, and collecting fluorescence signals.

Preferably, concentrations of the primers and probe in a reaction system are as follows respectively: the forward primers (F1/F3/F4/F5) have concentrations of 30 nM-1000 nM respectively, the reverse primer (R1/R3/R4/R5) have concentrations of 30 nM-500 nM respectively, and the detection probe (P1) has a concentration of 150 nM-1200 nM.

Compared with the prior art, the advantages of the technical solution described in the present invention lie in:
Ultra-multiplex detection: when the method described in the present invention is used, both of a fluorescence channel analysis and melting temperature analysis in a single tube reaction may be simultaneously performed in two dimensions, that is, different targets may be detected by using a same fluorescence channel and by means of melting temperature characteristics; or, by using different fluorescent channels, the detection of target species may be achieved by multiplying the number of the fluorescent channels by the melting temperature characteristics, for example, when two melting temperature detection points are set in one fluorescence channel, if there are two fluorescence channels, 2×2 target sequences may be detected, thereby achieving a multiplex detection of more target sequences; in addition, by arranging a blocking region on the detection probe, the amplification length of the secondary amplification product may be shortened, such that the detection efficiency may be improved, meanwhile, the melting peak values of different targets may be effectively set, and the resolution of melting temperatures (Tm values) may be improved, thereby further achieving ultra-multiplex detection.

Low fluorescence background: for each fluorescence channel, only one probe is used, and then a differentiation may be achieved by the different melting temperatures of the amplification products, thereby greatly reducing the fluorescence background in the PCR reaction and improving reaction sensitivity.

Adjustable melting temperature: in the method described in the present invention, two ends of the probe are simultaneously combined with two ends of the amplification product and then the probe is stretched and light is emitted, and thus, it is possible to increase or decrease the melting temperature of the double-stranded secondary amplification product formed with the detection probe (P) by adjusting a length or sequence of the sequence h, or adjusting a position at which the sequence h is reversely complementary to the detection sequence binding domain of the detection sequence binding region (H) of the detection probe (P).

Good inclusiveness: compared with a TaqMan Hydrolysis probe method which requires three parts that are complementarily paired with the template, in the primer probe design method of the present invention, only two parts, namely the forward primer (F) and the reverse primer (R) are complementarily paired with the target sequence, such that when there are many high variation regions in the target sequence (such as viruses or bacterial genomes), the primer probe of the present invention has a better inclusiveness and a lower design difficulty.

Low cost: by means of the method of the present invention, a detection may be completed in a single tube reaction by only requiring a fluorescence quantitative PCR instrument, without the need for additional downstream instruments and without the need for chips or multiple reaction tubes for dividing reactions, thereby greatly reducing the cost of instruments and consumables, meanwhile, the number of fluorescence probes in the reagent is reduced, thereby greatly reducing the cost of the reagent, and allowing a large-scale promotion to be possible.

Single tube reaction: the method has low sample consumption, is particularly suitable for the detection of a rare sample, and can greatly increase a concentration of the sample added to the detection reaction, thereby improving detection sensitivity, and providing the possibility for a subsequent extraction-free one-tube detection.

Easy and simple operation: only a fluorescence quantitative PCR instrument is required for detection, without subsequent steps such as for capillary electrophoresis or nucleic acid hybridization, etc., meanwhile, the preparation is simple, and there is no need for manual operation after the instrument is enabled.

No easy to cause pollution: in the method described in the present invention, the instrument is completely closed after the sample addition is completed, without the need to open the cover for subsequent detection, thereby greatly reducing the possibility of polluting an experimental environment.

High sensitivity: the method described in the present invention has a very low requirement for the length of the target sequence, when the target type is a short fragment nucleic acid, such as a free nucleic acid, a higher sensitivity is achieved in detection for a shorter target sequence length.

Wide applicable range: the present invention is suitable for nucleic acid detection of various sample types, including a serum sample, a plasma sample, a whole blood sample, a sputum sample, a swab sample, a lavage fluid sample, a fresh tissue sample, a formalin fixed paraffin embedded tissue (FFPE), and a target (such as bacteria, viruses, gene mutations), etc., such that clinical units, including primary hospitals, can quickly carry out a common target detection (such as a detection for pathogenic microorganism infection), which may be assisted with other detection and definite diagnostic means, thereby quickly providing diagnostic evidence and medication schemes for clinical practice, reducing mental and economic burden of patients, and achieving an objective of precision medicine.

The preferred embodiments of the present invention will be described below, but the protection scope of the present invention is not limited to the following preferred embodiments. It should be noted that, several variations and improvements made by those skilled in the art based on the concept of the present invention belong to the protection scope of the present invention. The used reagents without specifying the manufacturer are all conventional products that may be commercially available.

### Examples

### Example 1. Single detection for Human Papillomavirus HPV type 45

**Table 1: Probe primer**

| Name of primer probe | Number | Nucleotide sequence (5'-3') | Label |
|---|---|---|---|
| P1 | SEQ ID NO:1 | AGGACGTGACGTCTC-C3Spacer- TCAGCACGTAGCTCCTCGATGCCTGCGCT | C3 Spacer being labelled between |
| | | CCTGTACCTGACTGC | C15 and T16, T16-ROX, T28-BHQ2, 3'Spacer C3 |
| R1 | SEQ ID NO:2 | TCAGCACGTAGCTAAATCGTCGGGCTGGT AGTTG | / |
| F1 | SEQ ID NO:3 | ACGTCACGTCCTAAACGATGAAGCAGATG GCGTTA | / |

Referring to Table 1, the forward primer F1 (SEQ ID NO: 3) is a specific primer designed for a target sequence of human papillomavirus HPV type 45, and the F1 has a total length of 35 bp, wherein the 1^{st} to 20^{th} bases at 3' end thereof serve as a target sequence binding region, and the 1^{st} to 12^{th} bases at 5' end thereof serve as a sequence A, which is reversely complementary to a sequence of the 1^{st} to 12^{th} bases (i.e. a first binding region A') at 5' end of the probe P1 (SEQ ID NO: 1).

Referring to Table 1, the reverse primer R1 (SEQ ID NO: 2) is a specific primer designed for the target sequence of human papillomavirus HPV type 45, and the R1 has a total length of 34 bp, wherein the 1^{st} to 18^{th} bases at 3' end thereof serve as a target sequence binding region, the 1^{st} to 13^{th} bases at 5' end thereof serve as a sequence h (h), which is identical to a sequence of the 16^{th} to 28^{th} bases at 3' end of the probe P1 (SEQ ID NO: 1) (i.e., a FRET region of the probe).

The reaction system comprises the components described in Table 2 below:

**Table 2. Reaction system**

| Reagent components | | Concentration |
|---|---|---|
| Single detection system for human papillomavirus HPV type 45 | 2×PCR Reaction Buffer | 1× |
| | DNA Polymerase | 3U |
| | Probe (P1) | 400 nM |
| | Forward primer (F1) | 500 nM |
| | Reverse Primer (R1) | 100 nM |
| | Nucleic acid template | 50 copies |
| | Ultrapure water | Adding to 25 µ L |

The reaction buffer and a primer probe premixed solution were mixed according to the reaction system described in Table 2. The nucleic acid templates to be detected were human papillomavirus HPV type 45 plasmids synthesized by General Biological (Anhui) Co., Ltd.. The human papillomavirus HPV type 45 plasmids were quantified and diluted, and then mixed with the reaction system at a ratio of 50 copies/reaction. And at the same time, a no-template control (NTC) (obtained by replacing the nucleic acid template with ultrapure water or 1 × TE buffer) was prepared. Then a PCR amplification and a melting curve analysis were performed. The presence or absence of HPV type 45 in the sample may be judged based on a characteristic Tm value of the target in a specific detection channel.

The detection was performed by using SLAN real-time fluorescence quantitative PCR instrument and reagent consumables from Shanghai Hongshi Medical Technology Co., Ltd. Wherein, 2x PCR reaction buffer comprises: 3 mM MgCl₂, 30 mM Tris-HCl with a pH of 8.3, 0.5 mM dNTP, and 70 mM (NH₄)₂SO₄, etc.

The detection results of the reagent kit were subjected to data analysis by using an analysis software of the SLAN real-time fluorescence quantitative PCR instrument from Shanghai Hongshi Medical Technology Co., Ltd.

### Sample preparation:

The human papillomavirus HPV type 45 plasmids which were synthesized by General Biological (Anhui) Co., Ltd., were quantified and diluted, and then were used as positive control templates. A reverse primer (R) with a signal detection region sequence were used to separately detect the human papillomavirus HPV type 45, to verify a single detection ability. The ultrapure water or 1 × TE Buffer was used as a no template control (NTC).

### Reaction preparation:

After the sample preparation was completed, the reaction system was prepared according to the ratios described in Table 2.

PCR amplification and melting curve analysis:
After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of the fluorescence quantitative PCR instrument. The procedure used was as follows: performing pre-denaturation at 95°C for 3 minutes; performing denaturation at 94 °C for 10 seconds, and performing annealing and extension at 56 °C for 30 seconds, for a total of 45 cycles; and performing melting curve analysis at 40-85°C, with a heating rate of 0.05°C/s, and collecting fluorescence signals.

### Data analysis:

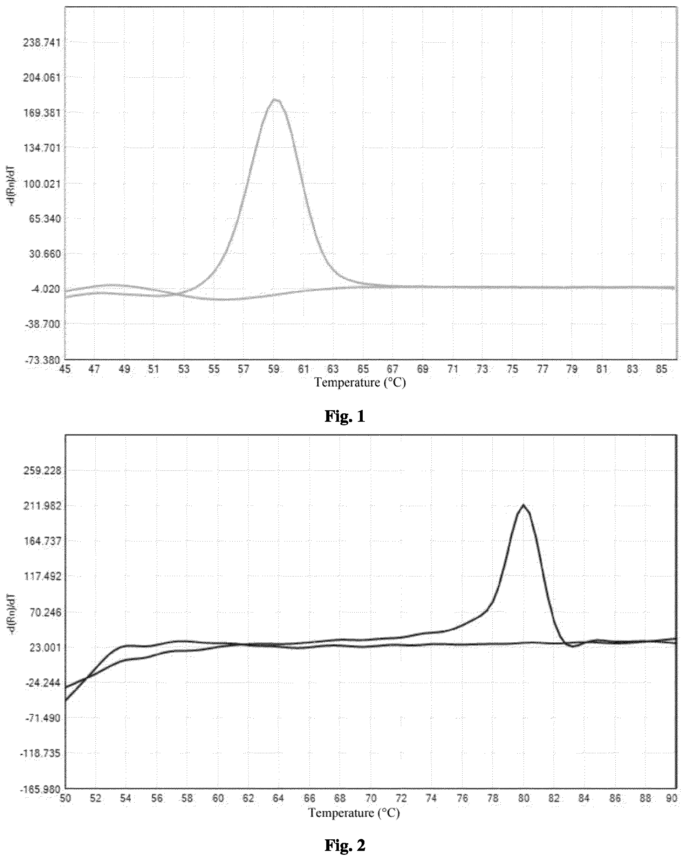

The results were interpreted via a Tm value of the melting curve. Fig. 1 shows that there is no melting peak for a no-template control, while a melting peak with a Tm value of 59°C is formed in a ROX channel for a positive reaction well added with HPV type 45 plasmids.

### Example 2. Single detection for Human Papillomavirus HPV type 16

**Table 3: Probe primer**

| Name of primer probe | Number | Nucleotide sequence (5'-3') | Label |
|---|---|---|---|
| P2 | SEQ ID NO:4 | CCTCTCCTACTTGGCA-BHQ- TCTCGATGACTGATCGCACAGTCCGTAC CCGATCGGCTGACGA | BHQ2 being labelled between A16 and T17, T30-FAM, 3' Spacer C3 |
| R2 | SEQ ID NO:5 | CGGCTGACGAAAAACGCACAACCGAAG CGTAGA | / |
| F2 | SEQ ID NO:6 | GCCAAGTAGGAGAGGAAAGAACCGGAC AGAGCCCATT | / |

Referring to Table 3, the forward primer F2 (SEQ ID NO: 6) is a specific primer designed for a target sequence of human papillomavirus HPV type 16, and the F2 has a total length of 37 bp, wherein the 1^{st} to 19^{th} bases at 3' end thereof serve as a target sequence binding region, and the 1^{st} to 15^{th} bases at 5' end thereof serve as a sequence A, which is reversely complementary to a sequence of the 1^{st} to 15^{th} bases (i.e. a first binding region A') at 5' end of the detection probe P2 (SEQ ID NO: 4).

Referring to Table 3, the reverse primer R2 (SEQ ID NO: 5) is a specific primer designed for the target sequence of human papillomavirus HPV type 16, and the R2 has a total length of 33 bp, wherein the 1^{st} to 19^{th} bases at 3' end thereof serve as a target sequence binding region, the 1^{st} to 10^{th} bases at 5' end thereof serve as a sequence h, which is identical to a sequence of a 1^{th} to 10^{th} bases at 3' end of the probe P1 (SEQ ID NO: 4).

The reaction system for human papillomavirus HPV type 16 detection comprises the components described in Table 4 below:

**Table 4: Reaction system**

| Reagent components | | Concentration |
|---|---|---|
| Single detection system for human papillomavir us type 16 | 2×PCR Reaction Buffer | 1× |
| | DNA Polymerase | 3U |
| | Probe (P2) | 400 nM |
| | Forward primer (F2) | 500 nM |
| | Reverse Primer (R2) | 100 nM |
| | Nucleic acid template | 50 copies |
| | ultrapure water | Adding to 25 µL |

The reaction buffer and a primer probe premixed solution were mixed according to the reaction system described in Table 4. The nucleic acid templates to be detected were human papillomavirus HPV type 16 plasmids synthesized by General Biological (Anhui) Co., Ltd.. The human papillomavirus HPV type 45 plasmids were quantified and diluted, and then mixed with the reaction system at a ratio of 50 copies/reaction. And at the same time, a no-template control (NTC) (obtained by replacing the nucleic acid template with ultrapure water or 1 × TE buffer) was prepared. Then a PCR amplification and a melting curve analysis were performed. The presence or absence of HPV type 16 in the sample may be judged based on a characteristic Tm value of the target in a specific detection channel.

The detection was performed by using SLAN real-time fluorescence quantitative PCR instrument and reagent consumables from Shanghai Hongshi Medical Technology Co., Ltd. The 2x PCR reaction buffer comprises: 3 mM MgCl₂, 30 mM Tris-HCl with a pH of 8.3, 0.5 mM dNTP, and 70 mM (NH₄)₂SO₄, etc.

The detection results of the reagent kit were subjected to data analysis by using an analysis software of the SLAN real-time fluorescence quantitative PCR instrument from Shanghai Hongshi Medical Technology Co., Ltd.

### Sample preparation:

The human papillomavirus HPV type 16 plasmids which were synthesized by General Biological (Anhui) Co., Ltd., were quantified and diluted, and then were used as positive control templates. A reverse primer (R) with a signal detection region sequence were used to separately detect the human papillomavirus HPV type 16, to verify a single detection ability. The ultrapure water or 1 × TE Buffer was used as a no template control (NTC).

### Reaction preparation:

After the sample preparation was completed, the reaction system was prepared according to the ratios described in Table 4.

PCR amplification and melting curve analysis:
After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of the fluorescence quantitative PCR instrument. The procedure used was as follows: performing pre-denaturation at 95°C for 3 minutes; performing denaturation at 94 °C for 10 seconds, and performing annealing and extension at 56 °C for 30 seconds, for a total of 45 cycles; and performing melting curve analysis at 50-90°C, with a heating rate of 0.05°C/s, and collecting fluorescence signals.

### Data analysis:

The results were interpreted via a Tm value of the melting curve. Fig. 2 shows that there is no melting peak for a no-template control, while a melting peak with a Tm value of 80°C is formed in a FAM channel for a positive reaction well added with HPV type 16 plasmids.

### Example 3: Multiplex detection for human papillomaviruses HPV types 16, 31, 35, and 45

**Table 5: Primer probe system**

| Name of primer probe | Number | Nucleotide sequence (5'-3') | Label |
|---|---|---|---|
| P1 | SEQ ID NO:1 | AGGACGTGACGTCTC-C3Spacer- TCAGCACGTAGCTCCTCGATGCCTGCG CTCCTGTACCTGACTGC | C3 Spacer being labelled between C15 and T16, T16-ROX, T28-BHQ2, 3'Spacer C3 |
| R1 | SEQ ID NO:2 | TCAGCACGTAGCTAAATCGTCGGGCTG GTAGTTG | / |
| F1 | SEQ ID NO:3 | ACGTCACGTCCTAAACGATGAAGCAG ATGGCGTTA | / |
| F3 | SEQ ID NO:7 | ACGTCACGTCCTAAAGGAGCGACCC AGAAAGT | / |
| R3 | SEQ ID NO:8 | ACCTGACTGCAAAGCTCTGTGCATAAC TGTGGT | / |
| F4 | SEQ ID NO:9 | ACGTCACGTCCTAAATGGATCAGCCAT TGTAGTTACAG | / |
| R4 | SEQ ID NO:10 | GCGCTCCTGTAAATGGAATCGTGTGCC CCAA | / |
| F5 | SEQ ID NO:11 | ACGTCACGTCCTAAACGCCTCACATTT ACAACAGGAC | / |
| R5 | SEQ ID NO:12 | CTCGATGCCTAAAACAAGCAAAACCA GACACCTC | / |

Referring to Table 5, the forward primers F1 (SEQ ID NO: 3), F3 (SEQ ID NO: 7), F4 (SEQ ID NO: 9), and F5 (SEQ ID NO: 11) are specific primers designed for target sequences of human papillomaviruses HPV type 45, 16, 31, and 35, respectively. The full lengths of the forward primers F1 (SEQ ID NO: 3), F3 (SEQ ID NO: 7), F4 (SEQ ID NO: 9), and F5 (SEQ ID NO: 11) are 35bp, 33bp, 38bp, and 37bp respectively, and all of the 1^{st} to 12^{th} bases at 5' ends thereof serve as sequences A, each of which is reversely complementary to a sequence of the 1^{st} to 12^{th} bases (i.e. a first binding region A') at 5' end of the probe P1 (SEQ ID NO: 1). The 1^{st} to 20^{th} bases at 3' end of the forward primer F1 (SEQ ID NO: 3) serve as a specific sequence for the target sequence of human papillomavirus HPV type 45, the 1^{st} to 17^{th} bases at 3' end of the forward primer F3 (SEQ ID NO: 7) serve as a specific sequence for the target sequence of human papillomavirus HPV type 16, the 1^{st} to 23^{rd} bases at 3' end of the forward primer F4 (SEQ ID NO: 9) serve as a specific sequence for the target sequence of human papillomavirus HPV 31, and the 1^{st} to 22^{nd} bases at 3' end of the forward primer F5 (SEQ ID NO: 11) serve as a specific sequence for the target sequence of human papillomavirus HPV 35.

Referring to Table 5, the reverse primers R1 (SEQ ID NO: 2), R3 (SEQ ID NO: 8), R4 (SEQ ID NO: 10), and R5 (SEQ ID NO: 12) are specific primers designed for target sequences of human papillomaviruses HPV type 45, 16, 31, and 35, respectively. The full lengths thereof are 34bp, 33bp, 31bp, and 34bp respectively. The 1^{st} to 13^{th} bases at 5' end of the R1 (SEQ ID NO: 2) serve as a sequence h (h), which is reversely complementary to a sequence of a 16^{th} to 28^{th} bases at 5' end of the probe P1 (SEQ ID NO: 1); and all of the 1^{st} to 10^{th} bases at 5' ends of R3 (SEQ ID NO: 8), R4 (SEQ ID NO: 10), and R5 (SEQ ID NO: 12) serve as sequences h, which have the same sequence as the 1^{st} to 10^{th}, 11^{th} to 20^{th}, 21^{st} to 30^{th} bases at 3' end of the probe P1 (SEQ ID NO: 1), respectively. The 1^{st} to 21^{st} bases at 3' end of the reverse primer R1 (SEQ ID NO: 2) serve as a specific sequence for the target sequence of human papillomavirus HPV type 45, the 1^{st} to 20^{th} bases at 3' end of the reverse primer R3 (SEQ ID NO: 8) serve as a specific sequence for the target sequence of human papillomavirus HPV type 16, the 1^{st} to 18^{th} bases at 3' end of the reverse primer R4 (SEQ ID NO: 10) serve as a specific sequence for the target sequence of human papillomavirus HPV 31, and the 1^{st} to 21^{st} bases at 3' end of the reverse primer R5 (SEQ ID NO: 12) serve as a specific sequence for the target sequence of human papillomavirus HPV 35.

The multiplex detection reaction system comprises the components shown in Table 6 below:

**Table 6: Reaction system**

| Reagent components | | Concentration |
|---|---|---|
| A multiplex detection system for human papillomavirus types 16, 31, 35, and 45 | 2×PCR Reaction Buffer | 1× |
| | DNA Polymerase | 5U |
| | Probe (P2) | 400 nM |
| | Forward primer (F1) | 500 nM |
| | Reverse Primer (R1) | 100 nM |
| | Forward primer (F3) | 500 nM |
| | Reverse Primer (R3) | 100 nM |
| | Forward primer (F4) | 500 nM |
| | Reverse Primer (R4) | 100 nM |
| | Forward primer (F5) | 500 nM |
| | Reverse Primer (R5) | 100 nM |
| | Nucleic acid template | 50 copies |
| | ultrapure water | Adding to 25 µL |

The reaction buffer and a primer probe premixed solution were mixed according to the reaction system described in Table 6. The nucleic acid templates to be detected were human papillomaviruses HPV types 16, 31, 35, and 45 plasmids synthesized by General Biological (Anhui) Co., Ltd.. The human papillomaviruses HPV types 16, 31, 35, and 45 plasmids were quantified and diluted, and then mixed with the reaction system at a ratio of 50 copies/reaction respectively. And at the same time, a no-template control (NTC) (obtained by replacing the nucleic acid template with ultrapure water or 1 × TE buffer) was prepared. Then a PCR amplification and a melting curve analysis were performed. The presence or absence of HPV type to be detected in the sample may be judged based on a characteristic Tm value of the target in a specific detection channel.

The detection was performed by using SLAN real-time fluorescence quantitative PCR instrument and reagent consumables from Shanghai Hongshi Medical Technology Co., Ltd. The 2x PCR reaction buffer comprises: 3 mM MgCl₂, 30 mM Tris-HCl with a pH of 8.3, 0.5 mM dNTP, and 70 mM (NH₄)₂SO₄, etc.

The detection results of the reagent kit were subjected to data analysis by using an analysis software of the SLAN real-time fluorescence quantitative PCR instrument from Shanghai Hongshi Medical Technology Co., Ltd.

### Sample preparation:

The human papillomaviruses HPV types 16, 31, 35, and 45 plasmids which were synthesized by General Biological (Anhui) Co., Ltd., were quantified and diluted, and then were used as positive control templates. The reverse primers (R) with a signal detection region sequence were used to detect the human papillomaviruses HPV types 16, 31, 35, and 45, to verify a multiplex detection ability. The ultrapure water or 1 × TE Buffer was used as a no template control (NTC).

### Reaction preparation:

After the sample preparation was completed, the reaction system was prepared according to the ratios described in Table 4.

PCR amplification and melting curve analysis:
After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of the fluorescence quantitative PCR instrument. The procedure used was as follows: performing pre-denaturation at 95°C for 3 minutes; performing denaturation at 94 °C for 10 seconds, and performing annealing and extension at 56 °C for 30 seconds, for a total of 45 cycles; and performing melting curve analysis at 40-85°C, with a heating rate of 0.05°C/s, and collecting fluorescence signals.

### Data analysis:

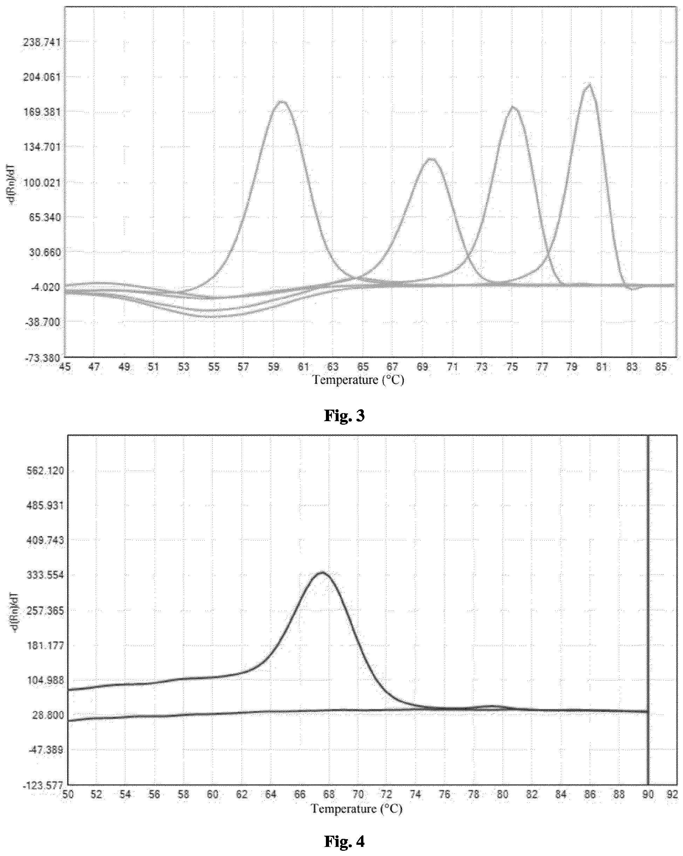

The results were interpreted via Tm values of the melting curves. Fig. 3 shows that there is no melting peak for a no-template control, while melting peaks with Tm values of 80.2°C, 75°C, 69.4°C, and 59.4°C are formed in a ROX channel for positive reaction wells added with HPV type 16, 31, 35, and 45 plasmids respectively.

### Example 4: Single detection for non-small cell lung cancer EGFR exon 20 H773_V774insH mutation

Primer probe system:

**Table 7: Primer probe**

| Name of primer probe | Number | Nucleotide sequence (5'-3') | Label |
|---|---|---|---|
| P3 | SEQ ID NO:13 | CCTGCGACGCTCATCTCAGTACAGC GTCTGTCGCTGATG-C3Spacer- CGCACACTGGGACCACCGCT | T31-CY5, T47-BHQ2 Spacer C3 between G39 and C40, 3' Spacer C3 |
| R6 | SEQ ID NO:14 | GACCACCGCTAAAAGTCTGAACGAC CTACACTCC | / |
| R7 | SEQ ID NO:15 | GTCGCTGATGAAAAGTCTGAACGAC CTACACTCC | / |
| F6 | SEQ ID NO:16 | GATGAGCGTCGCAGGTTTCCCAGCA GCTGACACA | / |

Referring to Table 7, the forward primer F6 (SEQ ID NO: 16) is a specific primer designed for a target sequence of non-small cell lung cancer EGFR exon 20 H773_V774insH mutation, and the F6 has a total length of 34 bp, wherein the 1^{st} to 16^{th} bases at 3' end thereof serve as a target sequence binding region, and the 1^{st} to 15^{th} bases at 5' end thereof serve as a sequence A, which is reversely complementary to a sequence of the 1^{st} to 15^{th} bases (i.e. a first binding region A') at 5' end of the probe P3 (SEQ ID NO: 13).

Referring to Table 7, both of the reverse primers R6-R7 (SEQ ID: 14-SEQ ID: 15) are all specific primers designed for the target sequence of the non-small cell lung cancer EGFR exon 20 H773_V774insH mutation, and the sequences of target binding region sequences thereof are identical. R6 has a total length of 34bp, wherein the 1^{st} to 21^{st} bases at 3' end thereof serve as a target sequence binding region, a sequence of the 1^{st} to 10^{th} bases at 5' end thereof is identical to a sequence of the 1^{st} to 10^{th} bases at 3' end of the probe P3 (SEQ ID NO: 13). R7 has a total length of 34bp, wherein the 1^{st} to 21^{st} bases at 3' end thereof serve as a target sequence binding region, a sequence of the 1^{st} to 10^{th} bases at 5' end thereof is identical to a sequence of the 21^{st} to 30^{th} bases at 3' end of the probe P3 (SEQ ID NO: 13).

**Table 8: Reaction system**

| Reagent components | | Concentration |
|---|---|---|
| Single detection system for non-small cell lung cancer EGFR exon 20 H773_V774i nsH mutation | 2x PCR Reaction Buffer | 1× |
| | DNA Polymerase | 2U |
| | Forward primer (F6) | 500 nM |
| | Reverse primer (R6 or R7) | 100 nM |
| | Nucleic acid template | 0.1 ng-60 ng |
| | Ultrapure water | Adding to 20 µL |

The reaction buffer and a primer probe premixed solution were mixed according to the reaction system described in Table 8, a nucleic acid was extracted from a sample to be detected by using an appropriate method and was added to the prepared reaction system. Then a PCR amplification and a melting curve analysis were performed. The type of pathogen in the sample may be judged based on a characteristic Tm value of the target in a specific detection channel.

The detection was performed by using SLAN real-time fluorescence quantitative PCR instrument and reagent consumables from Shanghai Hongshi Medical Technology Co., Ltd. The 2x PCR reaction buffer comprises: 3 mM MgCl₂, 30 mM Tris-HCl with a pH of 8.3, 0.5 mM dNTP, and 70 mM (NH₄)₂SO₄, etc.

Data analysis was performed by using an analysis software of the SLAN real-time fluorescence quantitative PCR instrument from Shanghai Hongshi Medical Technology Co., Ltd. The SLAN real-time fluorescence quantitative PCR instrument from Shanghai Hongshi Medical Technology Co., Ltd was adopted.

### Sample preparation:

A sample of non-small cell lung cancer EGFR exon 20 H773_V774insH mutation target sequence was used as a positive sample, and the reverse primers (R) with different signal detection region sequences were used to separately detect the non-small cell lung cancer EGFR exon 20 H773_V774insH mutation target sequence respectively, to verify a detection ability for single virus infection. The pure water was used as a no template control (NTC).

### Reaction preparation:

After the sample preparation was completed, the reaction system was prepared according to the ratios described in Table 8.

PCR amplification and melting curve analysis:
After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of the fluorescence quantitative PCR instrument. The procedure used was as follows: performing pre-denaturation at 95°C for 3 minutes; performing denaturation at 94 °C for 10 seconds, and performing annealing and extension at 56 °C for 30 seconds, for a total of 45 cycles; performing denaturation at 94 °C for 10 seconds, and performing annealing and extension at 40 °C for 15 seconds, for a total of 10 cycles; and performing melting curve analysis at 50-90°C, with a heating rate of 0.05°C/s, and collecting fluorescence signals.

### Data analysis:

The results were interpreted via Tm values of the melting curves.

The non-small cell lung cancer EGFR exon 20 was taken as an example, by arranging the detection sequence binding domains on the detection probe complementary to the detection sequence at different positions, the sequence of the downstream primers may be adjusted to achieve different melting curve melting peaks of the final amplification products (i.e. secondary amplification products) of the detection sequence with the detection probe.

Fig. 4 shows that there is no melting peak for a no-template control, while a melting peak with a Tm value of 67.62°C is formed in a CY5 channel for a positive sample reaction well added with non-small cell lung cancer EGFR exon 20 H773_V774insH mutation (F6 and R7 are used as the first primer set).

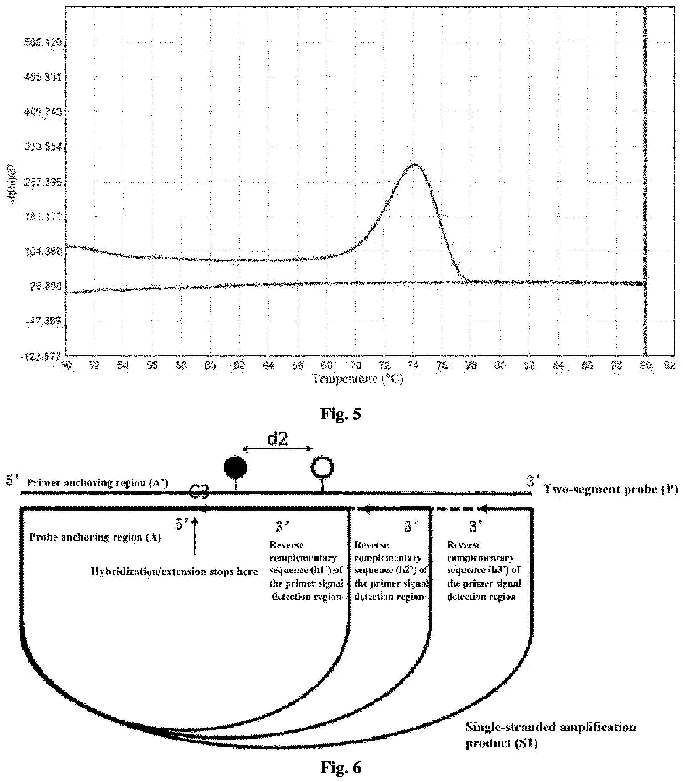

Fig. 5 shows that there is no melting peak for a no-template control, while a melting peak with a Tm value of 74.17°C is formed in a CY5 channel for a positive sample reaction well added with non-small cell lung cancer EGFR exon 20 H773_V774insH mutation (F6 and R6 are used as the first primer set).

### Comparative example: Comparison of multiplex detection between a probe comprising a blocking region and a probe comprising no blocking region

**Table 9: Primer probe system**

| Name of primer probe | Number | Nucleotide sequence (5'-3') | Label |
|---|---|---|---|
| P1 | SEQ ID NO:1 | AGGACGTGACGTCTC-C3Spacer- TCAGCACGTAGCTCCTCGATGCCTG CGCTCCTGTACCTGACTGC | C3 Spacer being labelled between C15 and T16, T16-ROX, T28-BHQ2, 3' Spacer C3 |
| P4 | SEQ ID NO:17 | AGGACGTGACGTCTCTCAGCACGTA GCTCCTCGATGCCTGCGCTCCTGTA CCTGACTGC | T16-ROX, T28-BHQ2, 3'Spacer C3 |
| R1 | SEQ ID NO:2 | TCAGCACGTAGCTAAATCGTCGGGC TGGTAGTTG | / |
| F1 | SEQ ID NO:3 | ACGTCACGTCCTAAACGATGAAGCA GATGGCGTTA | / |
| F4 | SEQ ID NO:9 | ACGTCACGTCCTAAATGGATCAGCC ATTGTAGTTACAG | / |
| R4 | SEQ ID NO:10 | GCGCTCCTGTAAATGGAATCGTGTG CCCCAA | / |
| F5 | SEQ ID NO:11 | ACGTCACGTCCTAAACGCCTCACAT TTACAACAGGAC | / |
| R5 | SEQ ID NO:12 | CTCGATGCCTAAAACAAGCAAAACC | / |
| | | AGACACCTC | |
| F7 | SEQ ID NO:18 | ACGTCACGTCCTAAAAAGG GCA ACG GCA TGG CA | / |
| R8 | SEQ ID NO:19 | ACCTGACTGCAAAGCA GTT TGT ATG GTC GTT GTG TA | / |

Referring to Table 9, the forward primers F1 (SEQ ID NO: 3), F4 (SEQ ID NO: 9), F5 (SEQ ID NO: 11), and F7 (SEQ ID NO: 18) are specific primers designed for target sequences of human papillomavirus types 45, 31, 35, and 59, respectively, wherein, the F1 (SEQ ID NO: 3), F4 (SEQ ID NO: 9), and F5 (SEQ ID NO: 11) are the same as those in Example 3 and will not be repeated. The forward primer F7 (SEQ ID NO: 18) has a total length of 33 bp, wherein the 1^{st} to 12^{th} bases at 5' end thereof serve as a sequence A, which is reversely complementary to a sequence of the 1^{st} to 12^{th} bases (i.e. a first binding region A') at 5' end of the detection probe P1 (SEQ ID NO: 1); and the 1^{st} to 16^{th} bases at 3' end thereof serve as a specific sequence for the target sequence.

Referring to Table 9, the reverse primers R1 (SEQ ID NO: 2), R4 (SEQ ID NO: 10), R5 (SEQ ID NO: 12), and R8 (SEQ ID NO: 19) are specific primers designed for target sequences of human papillomavirus types 45, 31, 35, and 59, wherein R1 (SEQ ID NO: 2), R4 (SEQ ID NO: 10), and R5 (SEQ ID NO: 12) are the same as those in Example 3 and will not be repeated. The reverse primer R8 (SEQ ID NO: 19) has a total length of 36bp, wherein the 1^{st} to 10^{th} bases at 5' end thereof serve as a sequence h (h), which is identical to a sequence of the 1^{st} to 10^{th} bases at 3' end of the detection probe P1 (SEQ ID NO: 1); and the 1^{st} to 23^{rd} bases at 3' end thereof serve as a specific sequence for the target sequence.

Two sets of multiplex reaction systems were set, wherein, in system ① the probe P1 (SEQ ID NO: 1), a probe comprising a blocking region, was used; in system ②, the P4 (SEQ ID NO: 17), a probe comprising no blocking region, was used; and the other components of the system were the same, as shown in Table 10. The detection performances of the two systems were compared.

**Table 10: Reaction system**

| Reagent components | | Concentration |
|---|---|---|
| Multiplex detection systems for human papillomavirus types 31, 35, 45, and 59 | 2×PCR Reaction Buffer | 1× |
| | DNA Polymerase | 5U |
| | Probe (P1/P4) | 400 nM |
| | Forward primer (F1) | 500 nM |
| | Reverse Primer (R1) | 100 nM |
| | Forward primer (F7) | 500 nM |
| | Reverse Primer (R8) | 100 nM |
| | Forward primer (F4) | 500 nM |
| | Reverse Primer (R4) | 100 nM |
| | Forward primer (F5) | 500 nM |
| | Reverse Primer (R5) | 100 nM |
| | Nucleic acid template | 50 copies |
| | Ultrapure water | Adding to 25 µ L |

For the system ① and system ②, the reaction buffer was mixed with a primer probe premixed solution as shown in Table 10. The nucleic acid templates to be detected were human papillomaviruses HPV types 31, 35, 45 and 59 plasmids synthesized by General Biological (Anhui) Co., Ltd.. The human papillomaviruses HPV types 31, 35, 45 and 59 plasmids were quantified and diluted, and then mixed with the reaction system at a ratio of 50 copies/reaction respectively. And at the same time, a no-template control (NTC) (obtained by replacing the nucleic acid template with ultrapure water or 1 × TE buffer) was prepared. Then a PCR amplification and a melting curve analysis were performed. The presence or absence of HPV type to be detected in the sample may be judged based on a characteristic Tm value of the target in a specific detection channel.

The detection was performed by using SLAN real-time fluorescence quantitative PCR instrument and reagent consumables from Shanghai Hongshi Medical Technology Co., Ltd. The 2x PCR reaction buffer comprises: 3 mM MgCl₂, 30 mM Tris-HCl with a pH of 8.3, 0.5 mM dNTP, and 70 mM (NH₄)₂SO₄, etc.

The detection results of the reagent kit were subjected to data analysis by using an analysis software of the SLAN real-time fluorescence quantitative PCR instrument from Shanghai Hongshi Medical Technology Co., Ltd.

### Sample preparation:

The human papillomaviruses HPV types 31, 35, 45 and 59 plasmids which were synthesized by General Biological (Anhui) Co., Ltd., were quantified and diluted, and then were used as positive control templates. The reverse primers (R) with a signal detection region sequence were used to detect the human papillomaviruses HPV types 31, 35, 45 and 59, to verify a multiplex detection ability. The ultrapure water or 1 × TE Buffer was used as a no template control (NTC).

### Reaction preparation:

After the sample preparation was completed, the reaction system was prepared according to the ratios described in Table 10.

PCR amplification and melting curve analysis:
After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of the fluorescence quantitative PCR instrument. The procedure used was as follows: performing pre-denaturation at 95°C for 3 minutes; performing denaturation at 94 °C for 10 seconds, and performing annealing and extension at 56 °C for 30 seconds, for a total of 45 cycles; and performing melting curve analysis at 40-85°C, with a heating rate of 0.05°C/s, and collecting fluorescence signals.

### Data analysis:

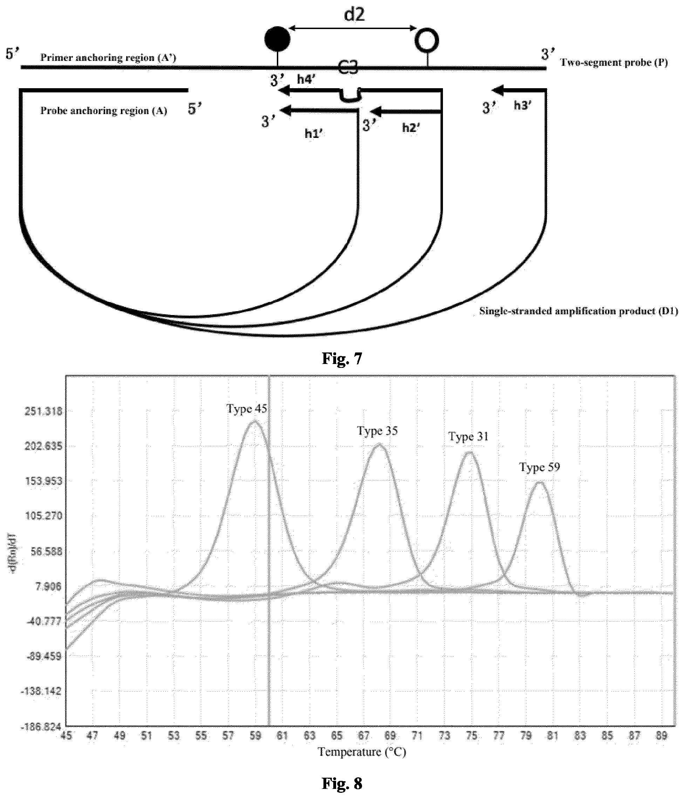

The results were interpreted via Tm values of the melting curves. Figure 8 shows a multiplex detection result of system ① (the probe comprising a blocking region), which shows that there is no melting peak for a no-template control, while melting peaks with Tm values of 59°C, 68.2°C, 75°C, and 80.2°C are formed in a ROX channel for positive reaction wells added with HPV types 45, 35, 31, and 59 plasmids respectively.

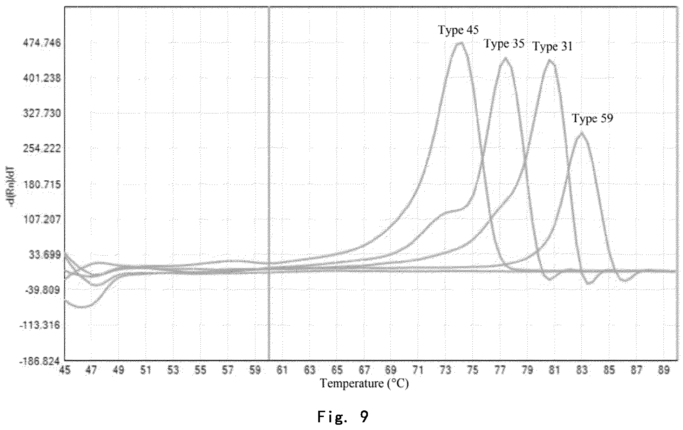

The results were interpreted via Tm values of the melting curves. Figure 9 shows a multiplex detection result of system ② (the probe comprising no blocking region), which shows that there is no melting peak for a no-template control, while melting peaks with Tm values of 74.2°C, 77.4°C, 80.6°C, and 83°C are formed in a ROX channel for positive reaction wells added with HPV types 45, 35, 31, and 59 plasmids respectively. The Tm differences between adjacent melting peaks are 3.2°C, 3.2°C, and 2.4°C, respectively.

## Claims

1. A composition for PCR detection of a target sequence, comprising a detection probe, the detection probe comprising a detection sequence binding region, a detection group, and a blocking region, wherein,
the detection sequence binding region is complementary to a detection sequence or a portion thereof,
the detection sequence binding region of the detection probe specifically binds to the detection sequence, followed by extending the detection sequence 0 or more nucleotides to obtain an amplification product of the detection sequence,
the detection sequence binding region does not contain a sequence complementary or identical to the target sequence,
when the binding site of the detection probe to the detection sequence is located downstream of the blocking region, the blocking region blocks further extension of the detection sequence, and
a detection group on the detection probe emits different signals under the condition of hybridization between the detection probe with the detection sequence or the amplification product thereof compared to the condition of non-hybridization between the detection probe with the detection sequence or the amplification product thereof.

2. The composition according to claim 1, wherein the detection sequence does not contain the target sequence or a portion thereof, or a sequence complementary to the target sequence or a portion thereof, and
the composition further comprises an auxiliary probe, the auxiliary probe comprising the detection sequence and a target specific sequence, wherein the target specific sequence comprises a sequence complementary to the target sequence or a portion thereof.

3. The composition according to claim 2, comprising n auxiliary probes and m detection probes for n target sequences, wherein 1 ≤ n ≤ 20, 1 ≤ m ≤ n,
preferably, the n auxiliary probes contain detection sequences that are different from each other,
preferably, each detection probe comprises a detection sequence binding region complementary to at least two types of detection sequences or a portion thereof, and the m detection probes at least comprise detection sequence binding domains complementary to n detection sequences or a portion thereof, and
preferably, the composition comprises n auxiliary probes and 1 detection probe for n target sequences, and the detection sequence binding region comprises ≥ n detection sequence binding domains for n detection sequences, wherein the individual detection sequence binding domains are arranged in a directly connected form, in a form connected via a connection sequence, or in a form with partial or complete overlapping.

4. The composition according to claim 3, further comprising n upstream oligonucleotide sequences for n target sequences respectively, wherein, the upstream oligonucleotide sequences each comprises a sequence complementary to a corresponding target sequence; and the upstream oligonucleotide sequences, after being hybridized with the target sequences, each is located at an upstream distal end of the auxiliary probe, or is located adjacent to an upstream of the auxiliary probe, or has an sequence partially overlapped with the target specific sequence of the auxiliary probe, and
preferably, the upstream oligonucleotide sequences are upstream primers specific to the target sequences or upstream probes specific to the target sequences.

5. The composition according to claim 4, further comprising n downstream oligonucleotide sequences for n target sequences respectively, wherein the downstream oligonucleotide sequences each comprises a sequence complementary to a corresponding target sequence; and, when being hybridized with the target sequences, the downstream oligonucleotide sequences each is located downstream of the target specific sequence.

6. The composition according to claim 1, wherein the detection sequence comprises a target sequence or a portion thereof, or a sequence complementary to the target sequence or a portion thereof,
the composition comprises a detection probe and a first primer set, wherein the first primer set comprises a first primer and a second primer that are each other's forward primer and reverse primer,
the detection probe comprises a first binding region A', a detection sequence binding region H, and a first blocking region; if the binding site of the detection probe to the detection sequence is located downstream of the first blocking region, the first blocking region blocks extension of the detection sequence,
preferably, the first primer comprises a sequence A that is partially or completely complementary to the first binding region A' of the detection probe, and the second primer comprises a sequence h that is partially or completely identical to the detection sequence binding region H of the detection probe,
the first binding region A', the detection sequence binding region H, the sequence A and the sequence h each is not complementary or identical to the target sequence or any fragment thereof, and
preferably, a detection group on the detection probe is located downstream of the first blocking region.

7. The composition according to claim 6, wherein the detection probe comprises:
one detection sequence binding region H and one first binding region A'; or
two detection sequence binding regions H and one first binding region A', the two detection sequence binding regions H being located on two sides of the first binding region A' respectively; or
one detection sequence binding region H and two first binding regions A', the two first binding regions A' being located on two sides of the detection sequence binding region H respectively; or
two detection sequence binding regions H and two first binding regions A' that are arranged at intervals.

8. The composition according to claim 6, comprising m detection probes and n first primer sets for n target sequences, wherein 1 ≤ n ≤ 20, 1 ≤ m ≤ n,
and the m detection probes at least comprise detection sequence binding domains complementary to n detection sequences or a portion thereof, and
wherein the individual detection sequence binding domains are arranged in a directly connected form, in a form connected via a connection sequence, or in a form with partial or complete overlapping.

9. The composition according to claim 6, wherein the detection probe is provided with a second blocking region at a 3' end thereof to prevent extension of the 3' end of the detection probe.

10. The composition according to claim 6, wherein the first primer comprises the sequence A and a target sequence binding region in sequence from 5' end to 3' end; and
the second primer comprises the sequence h and a target sequence binding region in sequence from 5' end to 3' end.

11. The composition according to claim 6, wherein a probe-primer set comprises m detection probes and n first primer sets for n target sequences, wherein 1 ≤ n ≤ 20, and 1 ≤ m ≤ n,
preferably, the probe-primer set comprises one detection probe; and detection sequences obtained by amplifying different target sequences with all of the first primer sets are hybridized with the detection probe, so as to be amplified to obtain amplification products, wherein annealing temperatures of hybrids formed by the amplification products and the detection probe are different from each other,
preferably, the detection sequences obtained by amplifying different target sequences with all of the first primer sets are hybridized with the detection probe, so as to be amplified to obtain amplification products, wherein annealing temperatures of hybrids formed by the amplification products and the detection probe differ from each other by ΔTₘ=6-10°C, and
preferably, all the target sequence binding regions included in the first primers are different from each other, and/or all the target sequence binding regions included in the second primers are different from each other.

12. A reaction system, comprising a composition of any one of claims 1-11.

13. A detection kit, comprising a composition of any one of claims 1-11 or a reaction system of claim 12.

14. A detection method, comprising a step of performing PCR using the composition of any one of claims 1-11, the reaction system of claim 12, or the detection kit of claim 13.

15. The method according to claim 14, comprising:
(1) reacting, under a condition that allows for hybridization, a detection sample with the upstream oligonucleotide sequence and the auxiliary probe;
(2) reacting, under a condition that allows for cleavage of the auxiliary probe, a product obtained in step (1) with an enzyme having 5' endonuclease activity and/or 5' exonuclease activity;
(3) reacting, under a condition that allows for hybridization, a product obtained in step (2) with the detection probe;
(4) reacting, under a condition that allows for extension reaction of a nucleic acid polymerase, a product obtained in step (3) with the nucleic acid polymerase;
(5) performing melting curve analysis to a product obtained in step (4); and determining the presence or absence of target sequence in the detection sample, based on a result of the melting curve analysis; wherein
preferably, under the condition that allows for nucleic acid hybridization, the detection sample is allowed to react with the upstream oligonucleotide sequence, the auxiliary probe, and the downstream oligonucleotide sequence; wherein the target sequence is used as a template, the upstream oligonucleotide sequence and downstream oligonucleotide sequence are used as an upstream primer and a downstream primer, respectively, for amplification, and
preferably, n upstream primers, n downstream primers, and n auxiliary probes are hybridized with corresponding n target sequences, respectively; subsequently, under action of the nucleic acid polymerase, all the upstream primers and downstream primers are extended respectively, and the extension of the upstream primers result in that corresponding auxiliary probes are cleaved by the enzyme having 5' endonuclease activity and/or 5' exonuclease activity, so as to release auxiliary fragments; then, the auxiliary fragments are hybridized to different positions of the detection probe and are extended by the nucleic acid polymerase, so as to obtain n extension products; and the n extension products and the detection probes form n hybrids with different Tm values.

16. The method according to claim 14, a first primer set is utilized to specifically bind with the target sequence and extend to produce a double-stranded pre-amplification product, wherein one single-stranded pre-amplification product of the double-stranded pre-amplification products serves as the detection sequence, and the detection sequence comprises the sequence A, the target sequence, and a complementary sequence h' of the sequence h; and
under a non-hybridization condition and a hybridization condition of the detection probe and the detection sequence, or the detection probe and the amplification product of the detection sequence, a signal change that is detected by an instrument is produced to determine the presence of the target sequence.

17. The method according to claim 16, the method comprises allowing the first primer and the second primer to bind with the target sequence respectively and extend to produce a double-stranded pre-amplification product, wherein one single-stranded pre-amplification product of the double-stranded pre-amplification product serves as the detection sequence, and the detection sequence comprises the sequence A, the target sequence, and a complementary sequence h' of the sequence h; the detection sequence binding region H of the detection probe is complementarily paired with the complementary sequence h' of the detection sequence h, and the first binding region A' of the detection probe is complementarily paired with the sequence A of the detection sequence; a 3' end of the detection sequence can continue to extend by 0 or more nucleotides to the 5' end of the detection probe (P) or the first blocking region, to obtain a partially or completely reverse complementary sequence of the detection probe (P) as an amplification product of the detection probe (P) and the detection sequence.

18. The method according to claim 16, wherein the signal change refers to a change in fluorescence signal or a change in melting temperature curve; preferably, the signal change refers to the change in melting temperature curve.

19. Use of the probe-primer set of any one of claims 1-11, the reaction system of claim 12, the detection kit of claim 13, or the detection method of claim 14 in PCR detection.

20. Use of claim 19, in performing single-tube ultra-multiplex PCR or digital PCR detection.

21. Use of the probe-primer set of any one of claims 1-11, the reaction system of claim 12, the detection kit of claim 13, or the method of any one of claims 14-18 in detection of clinically common pathogenic microorganism, non-invasive screening for birth defect, single nucleotide polymorphism analysis, methylation analysis, genetic mutation analysis, or genotyping.
